# EUROPEAN PATENT SPECIFICATION

(11) **EP 3 388 836 B1**
(45) Date of publication and mention of the grant of the patent: **25.11.2020**
(21) Application number: 17166358.6
(22) Date of filing: 12.04.2017
(51) Int. Cl.: G01N 33/574

(54) **METHODS FOR PREDICTING SURVIVAL AND TREATMENT SUSCEPTIBILITY OF GLIOBLASTOMA MULTIFORME (GBM) PATIENTS USING CYTOKINE PROFILES**
VERFAHREN ZUR VORHERSAGE DES ÜBERLEBENS UND DER BEHANDLUNGSSUSZEPTIBILITÄT VON PATIENTEN MIT GLIOBLASTOMA MULTIFORME (GBM) MITTELS CYTOKIN PROFILIERUNG
PROCÉDÉS PERMETTANT DE PRÉDIRE UNE SURVIE ET LA SUSCEPTIBILITÉ AU TRAITEMENT DE PATIENTS ATTEINTS DE GLIOBLASTOME MULTIFORME (GBM) EN UTILISANT LE PROFILE DE CYTOKINES

(43) Date of publication of application: 17.10.2018
(73) Proprietor: polybiocept GmbH, 63755 Alzenau (DE)
(72) Inventor: Mäurer, Markus, 184 44 Äkersberga (SE); Dodoo, Ernest, 112 34 Stockholm (SE); Geyer, Jakob, 28237 Bremen (DE)
(74) Representative: Patent- und Rechtsanwälte Ullrich & Naumann

(56) References cited:
- AFSAR RAHBAR ET AL: "Enhanced neutrophil activity is associated with shorter time to tumor progression in glioblastoma patients", ONCOIMMUNOLOGY, vol. 5, no. 2, 24 August 2015 (2015-08-24) , page e1075693, XP055387564, DOI: 10.1080/2162402X.2015.1075693
- EMMA SANDÉN ET AL: "Preoperative systemic levels of VEGFA, IL-7, IL-17A, and TNF-? delineate two distinct groups of children with brain tumors : Sand?n et?al.", PEDIATRIC BLOOD AND CANCER, vol. 63, no. 12, 29 July 2016 (2016-07-29) , pages 2112-2122, XP055387427, US ISSN: 1545-5009, DOI: 10.1002/pbc.26158
- ERNEST DODOO ET AL: "Expression of TAAs in glioblastoma and expansion of anti-TAA -reactive T cells", JOURNAL FOR IMMUNOTHERAPY OF CANCER, BIOMED CENTRAL LTD, LONDON, UK, vol. 2, no. Suppl 3, 6 November 2014 (2014-11-06), page P24, XP021202554, ISSN: 2051-1426, DOI: 10.1186/2051-1426-2-S3-P24
- MERLO A ET AL: "Cytokine gene expression in primary brain tumours, metastases and meningiomas suggests specific transcription patterns", EUROPEAN JOURNAL OF CANCER, ELSEVIER, AMSTERDAM, NL, vol. 29, no. 15, 1 January 1993 (1993-01-01), pages 2118-2125, XP026212521, ISSN: 0959-8049, DOI: 10.1016/0959-8049(93)90046-I [retrieved on 1993-01-01]

## Description

### FIELD OF THE INVENTION

The present invention relates to methods for predicting prolonged survival and treatment susceptibility of brain cancer patients and in particular to methods for predicting prolonged survival and increased treatment susceptibility of glioblastoma multiforme (GBM) patients. Embodiments of the invention have been particularly developed for identifying GBM patients with particular cytokine profiles in peripheral blood cerebrospinal fluid (CSF), bone marrow or tumour infiltrating lymphocytes (TIL) samples obtained prior to tumour surgery and will be described hereinafter with reference to this application.

### BACKGROUND OF THE INVENTION

Any discussion of the background art throughout the specification should in no way be considered as an admission that such art is widely known or forms part of common general knowledge in the field.

In 2014, the World Health Organisation estimated that circa 2% of all human cancers occur in the central nervous system (CNS). Although significantly less frequent than other solid tumours, such as lung cancer, melanoma, pancreatic cancer, CNS cancers are associated with very poor prognosis.

Glioblastoma, the most frequent and malignant primary brain tumour, stands apart from other neoplasms by its biology and location within the central nervous system (CNS). In spite of aggressive multimodal treatment including the three pillars of tumour surgery, radiotherapy and cytotoxic chemotherapy, the disease remains incurable. Commonly observed forms of glioma include i) diffuse or anaplastic astrocytoma (isocitrate dehydrogenase (IDH)-wild type/-mutant/not otherwise specified (NOS)); ii) oligodendroglioma or anaplastic oligodendroglioma (IDH-mutant and 1p/19q-codeleted/NOS); iii) oligoastrocytoma or anaplastic oligoastrocytoma (NOS) and iv) glioblastoma multiforme (GBM, IDH-wild type/-mutant/NOS).The most common and aggressive clinical manifestation of glioma is glioblastoma multiforme (GBM), which has a 5-year survival rate of less than 3%, as compared to the other primary gliomas listed here, which have a 5-year survival rate of at least 50%.

Patients diagnosed with primary GBM (IDH-wild type) represent approximately 90% of clinical cases, while those with secondary GBM (IDH-mutant) constitute the remaining 10%. The alkylating agent temozolomide (TMZ) is used as a first-line chemotherapeutic for the treatment of GBM, sometimes in combination with adjunctive immunotherapy.

One of the historic paradigms was that the nervous system was an immune privileged organ, devoid of normal immunologic function, a theory supported by the observations that the blood brain barrier only allows for selective entry of immune cells from the peripheral blood into the brain parenchyma, that the CNS lacks lymphatic vessels and lymph nodes, and that only low numbers of circulating T cells are found in the CNS.

The immune system recognises foreign and self antigens differentially. However, some foreign antigens are, by chance, highly similar to self antigen. Therefore, mechanisms have evolved that allow the immune system to regulate itself, such as to suppress certain immune responses thereby generally preventing autoimmunity. For example, in the event of T cell activation, membrane cytotoxic T-lymphocyte associated antigen 4 (CTLA4) and program cell death 1 (PD-1) proteins are up-regulated to inhibit continued T cell activation. Further, Regulatory T cells (Tregs) provide for a key regulatory mechanism in tumour tolerance and have a unique expression profile of cell surface markers including CD4 and CD25 (the alpha chain of the interleukin-2 (IL-2) receptor), CTLA4 8 and glucocorticoid-induced tumour necrosis factor (GITR) receptor. Treg production is linked to up-regulation of transcription factor called forkhead box protein 3 (FOXP3). Tregs actively inhibit CD4+ T cells, CD8+ T cells, dendritic cells and natural killer cells, and can, thereby, lead to suppression of immune responses - of undesirable autoimmune responses but also of immune responses against tumour cells in the tumour microenvironment. Other immune-regulatory mechanisms such as establishing a profile of immune-suppressive cytokines, production of myeloid-derived suppressor cells, regulatory B cells and natural killer cells play important roles in establishing and maintaining tumour tolerance. Accordingly, these mechanisms are considered targets of immune therapy of cancers including GBM.

For example, sporadic CNS inflammation has been attributed to GBM tumourigenesis, with the involvement of pro-inflammatory and pleiotropic cytokines such as interferon gamma (IFN-γ), tumour necrosis factor alpha (TNF-α), interleukin (IL) 6, IL-8, and IL-17A. As indicated above, it has been recognised that a key factor contributing to reduced survival of patients with GBM is the immunosuppressive tumour microenvironment, including Treg induction and increased PD-1 expression. Productive immune responses - directed against nominal tumour-associated antigens (TAA) are likely subdued in the GBM tumour microenvironment with the production of immune-tolerising cytokines playing a critical role in the balance of anti-tumour and tumourigenic responses. In addition, the administration of TMZ as the first-line chemotherapeutic agent contributes to local immune suppression by affecting tumour-infiltrating lymphocyte (TIL) numbers. As such, these immunosuppressive effects promote tumour progression in GBM patients. Other clinical parameters such as older age, exposure to cytotoxic chemotherapy and exogenous administration of corticosteroids are likely to contribute, and possibly potentiate, the immunosuppressive effects seen in GBM patients.

In Rahbar *et al.* (2015), the cytokine patterns in the blood of GBM patients with and without anti-viral treatment were characterised. It was determined whether neutrophil activation could be correlated to patient outcome. It was found that out of 28 patients, neutrophil activity was enhanced in 12 patients, who also displayed significantly shorter time to tumour progression.

In Sandén *et al.* (2016), 20 cytokines were analysed with respect to their applicability as indicator of patient outcome in cancer treatment. In particular, the study used a panel of four cytokines, namely VEGFA, IL-7, IL-17A and TNF-β, for the prediction of the patient outcome.

Dodoo *et al.* (2014) discloses that TAA-reactive T cells were identified by intracellular cytokine staining for IL-2, TNF, IFN and IL-17 and that TAA-reactive T cells could be successfully expanded *ex vivo.*

Merlo *et al.* (1993) describes determining cytokine gene expression profiles in primary brain tumour patients, including patients suffering from GBM. In particular, the expression of IL-4, IL-5 and IL-6 genes was investigated in brain tumour samples as well as in autologous peripheral blood mononuclear cells (PBMCs) of those patients.

In light of the relative immune-privilege of the brain and the immune-suppression caused by high grade gliomas, GBM immunotherapy has unique challenges in the CNS. Notwithstanding, reducing or reversing immunosuppressive effects to allow for an effective immune targeting of GBM would be highly desirable and various immunotherapeutic approaches are currently being investigated.

Generally, immunotherapy can be classified into broad groups, passive and active immunotherapy. Passive immunotherapy is based on the adoptive transfer of immunomodulators including cytokines, tumour specific antibodies or immune cells. These substances or cells are then administered to the patient to initiate an anti-tumour action. In general these therapies do not generate immunologic memory and therefore require chronic infusion based treatment. Active immunotherapies, on the other hand, stimulate the patient's immune system with the intent of promoting an antigen specific anti-tumour effect using the body's own immune cells. In addition active immunotherapies seek to create durable anti-tumour response that can protect against minimal residual disease and tumour recurrences.

For example, passive immunotherapy includes the administration of antibodies directed against tumour cells and does not require the patient's immune system to produce such antibodies. One such immunotherapy adjunctive to the 3-pillar GBM therapy is the administration of the anti-vascular endothelial growth factor (VEGF) antibody, bevacizumab (Avastin®). In cell-based passive immunotherapy approaches, immune cells specifically stimulated and activated for tumour recognition *in vitro* are administered to the patient. Such immunotherapy approaches are generally referred to as adoptive cell therapies and can include the transfusion of lymphocytes using cells from different origins such as tumour infiltrating lymphocytes (TIL), or peripheral blood mononuclear cells (PBMCs) stimulated by antigens, or chimeric antigen receptors CARs directed against tumour tissue. Currently ongoing investigations of adoptive cell therapies for GBM include the administration of T cells endowed with chimeric antigen receptors (CARs) targeting TAAs such as Epidermal Growth Factor Receptor variant III (EGFRvIII; clinical trials identifier: NCT02209376), IL-13R alpha 2, or Her2 (clinical trials identifier: NCT02442297). Notwithstanding, developing clinically-effective and commercially-viable adoptive cell therapies is being considered as one of the great challenges in advancing personalised medicine.

Notwithstanding, clinically-relevant and a long-term remission using T cells directed against tumours (tumour reactive T cells) have also been achieved in patients with melanoma.

Peptide-based active immunotherapy constitutes a vaccination against the tumour by injection of peptides resembling tumour-specific or tumour-associated antigens. These antigens can be categorised as cancer-testis (CT) antigens, tumour differentiation antigens, virus-related antigens, or and actions resulting from mutated oncogenic proteins. Peptides considered suitable for use as cancer vaccines are typically small, around nine amino acids in length and are capable of binding to major histocompatibility complex (MHC) class I molecules. EGFRvIII is a tumour-specific antigen commonly but exclusively expressed by glioblastoma cells, which has been the focus of intense investigation in relation to peptide-based active immunotherapy. In particular, PEP-3, a 14-mer peptide of EGFRvIII conjugated to keyhole limpet hemocyanin (PEP-3-KHL) has been considered a promising vaccine candidate in several clinical studies.

In cell-based active immunotherapy, i.e. cell-based vaccination, antigen-presenting cells activated by tumour antigens are being used to prime the immune system. In most cases, the antigen-presenting cells are prepared from autologous peripheral blood mononuclear cells (PBMCs), are cultivated in the presence of growth factors, and are then matured and activated with selected tumour-specific or tumour-associated prior to administration to the patient.

Accordingly, there is an urgent need for methods of predicting a GBM patient's individual prognosis and susceptibility to GBM therapies, including immunotherapies, such as to allow for the development of personalised GBM treatment plans, where such plans can tailor new adjunctive immunotherapeutic agents to the individual patient's immune profile as well as , the identification of patients most-likely to benefit from immunotherapies, thereby improving clinical outcomes and increasing overall survival of GBM patients.

It is an object of the present invention to overcome or ameliorate at least one of the disadvantages of the prior art, or to provide a useful alternative. In particular, it is an object of the present invention to provide improved methods for predicting a GBM patient's chance for prolonged survival following GBM surgery and therapy and for predicting a GBM patient's susceptibility to immunotherapy.

### SUMMARY OF THE INVENTION

The present invention relates to methods for predicting prolonged survival and/or susceptibility to immunotherapy of individual glioblastoma multiforme (GBM) patients. The embodiments of the present invention are based on the unexpected finding that an individual GBM patient's cytokine profile determined prior to tumour surgery can serve as highly reliable predictor for the individual patient's survival following GBM tumour surgery and GBM therapy.

Accordingly, in a first aspect the present invention relates to a method for the prediction of prolonged survival of a glioblastoma multiforme (GBM) patient, wherein the method comprises determining the cytokine profile in a previously-obtained sample of the GBM patient's peripheral blood, cerebrospinal fluid (CSF), bone marrow or tumour infiltrating lymphocytes (TIL), wherein the identification of all or none of either:
(a) interleukin-4 (IL-4), interleukin-5 (IL-5) and interleukin-6 (IL-6); or
(b) interferon gamma (IFN-γ), tumour necrosis factor alpha (TNF-a) and interleukin-17A (IL-17A),
in the patient's cytokine profile is indicative of the patient's prolonged survival following GBM therapy.

In a second aspect, the present invention relates to a method of predicting a GBM patient's increased susceptibility to immunotherapy, wherein the method comprises determining the cytokine profile in a previously-obtained sample of the GBM patient's peripheral blood, cerebrospinal fluid (CSF), bone marrow or tumour infiltrating lymphocytes (TIL), wherein the identification of all or none of either:
(a) interleukin-4 (IL-4), interleukin-5 (IL-5) and interleukin-6 (IL-6); or
(b) interferon gamma (IFN-γ), tumour necrosis factor alpha (TNF-a) and interleukin-17A (IL-17A),
in the patient's cytokine profile is indicative of the patient's increased susceptibility to immunotherapy.

### FIGURES

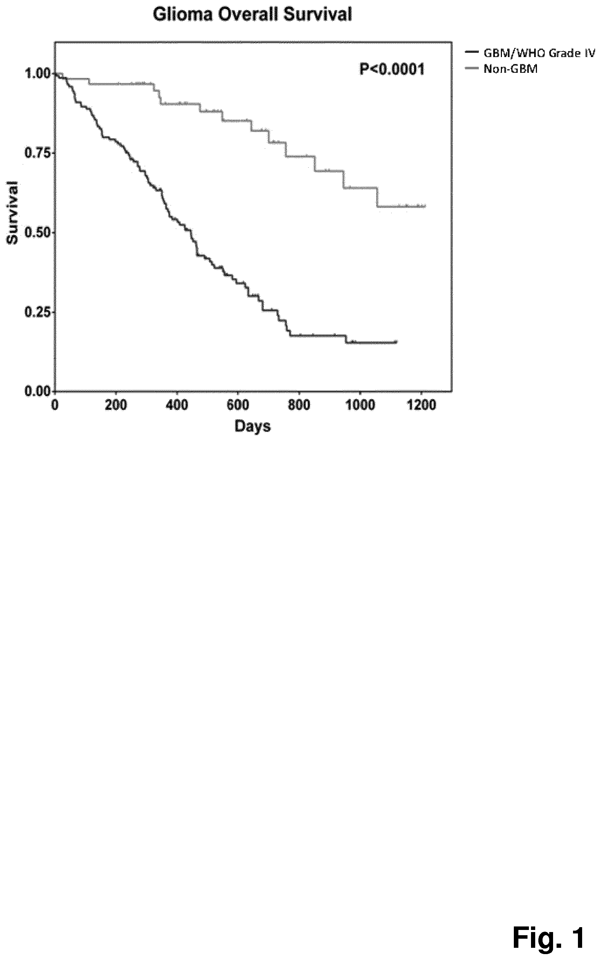

- Fig. 1: **shows overall survival pattern of patients with malignant glioma in the study cohort.** Patients with different diagnoses of malignant glioma were clinically followed up for at least 1200 days (40 months) post-surgery. Kaplan-Meier survival analyses were performed after segregating patients into two groups: patients with GBM/WHO CNS tumour grade IV malignant glioma or patients with non-GBM malignant glioma (WHO CNS tumour grades I-III)

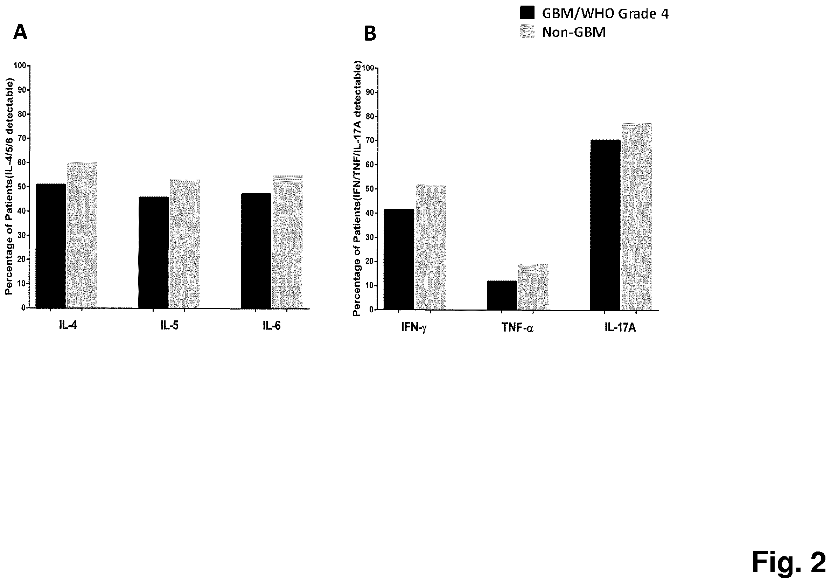

- Fig. 2: **shows anti-inflammatory/pleiotropic as well as pro-inflammatory/Th1 cytokine levels in serum of patients with malignant glioma.** Sandwich ELISA was used for measuring levels of (A) cytokines designated as anti-inflammatory or pleiotropic (IL-4, IL-5 and IL-6), as well as (B)cytokines with pro-inflammatory or Th1 attributes (IFN-γ, TNF-α and IL-17A), in the sera of patients with glioma (GBM and non-GBM) and expressed in pg/ml. The patients per glioma classification used in this study (GBM vs. non-GBM) with detectable amounts of cytokine (per detection limit of the individual cytokine ELISA assays) were presented as a fraction (in percentages) of the total number of individuals in the group (GBM=145 patients; non-GBM=60 patients). The GBM and non-GBM group of patients exhibited a similar pattern in terms of cytokine levels in serum.

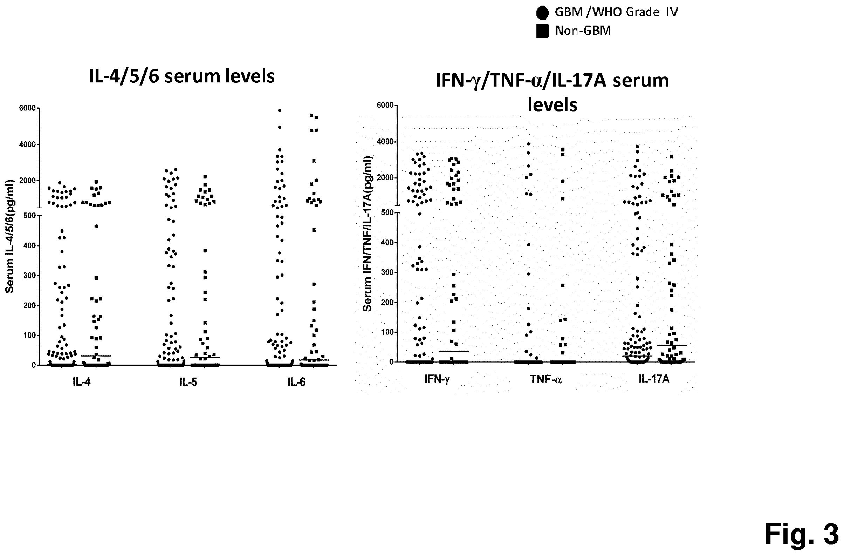

- Fig. 3: **shows anti-inflammatory/pleiotropic as well as pro-inflammatory/Th1 cytokine levels in sera of individual patients with malignant glioma.** Serum levels of IL-4, IL-5, IL-6, IFN-γ, TNF-α and IL-17A were measured by sandwich ELISA, and individual concentrations of cytokine levels (pg/ml) among patients with GBM vs. non-GBM are shown. As depicted in the Figure, the medians between the two patient groups across all the cytokines tested did not differ significantly, although there appears to be a trend toward meagrely higher levels among patients with non-GBM malignant glioma (except for TNF-α).

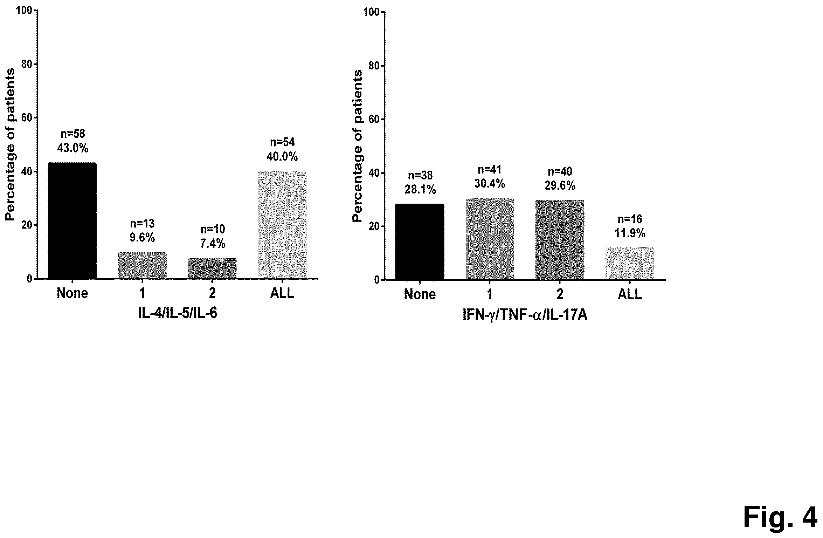

- Fig. 4: **shows serum cytokine profiles in patients with GBM.** Based on the actual concentration of cytokines measured by ELISA, patients were categorised for the survival analysis based on the detection of none, one, two or three cytokines (belonging to the 'anti-inflammatory/pleiotropic' or 'pro-inflammatory/Th1' grouping system) in serum. The number of patients ascribed to each category as well as the relative proportion to the total number of patients with GBM (%) is shown. Left panel, IL-4/IL-5IL-6; right panel, lFN-γ/TNF-α/lL-17A.

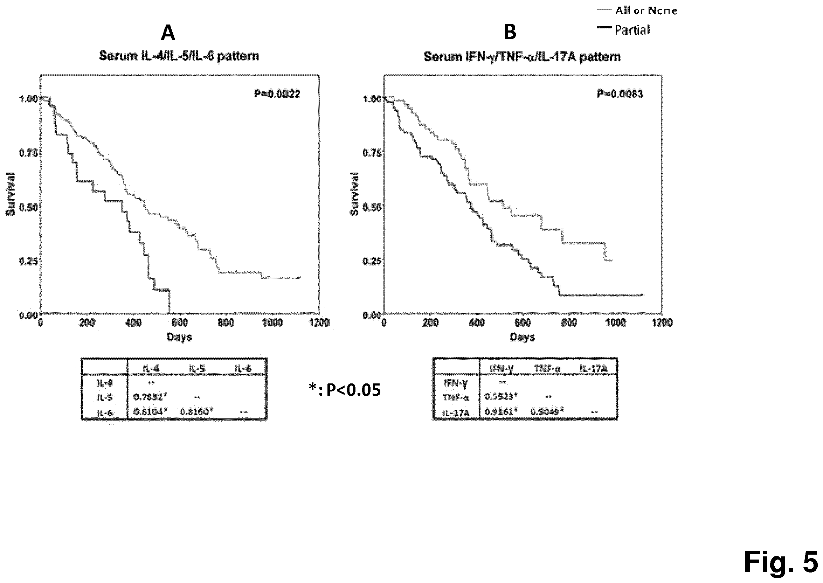

- Fig. 5: **shows the combinatorial effect of serum cytokines on the survival pattern of patients with GBM based on univariate analysis.** Kaplan-Meier survival analyses showing the combined effect of the two functional networks of serum cytokines (IL-4/IL-5/IL-6 and IFN-γ/TNF-α/IL-17A) on the survival pattern of patients with GBM up to 1200 days post-surgery. Having all or none of the cytokines as opposed to only partial combinations (one or two cytokines) in each functional network was correlated with improved survival of patients. The interaction between individual cytokines in the two functional networks was also ascertained using Spearman's correlation

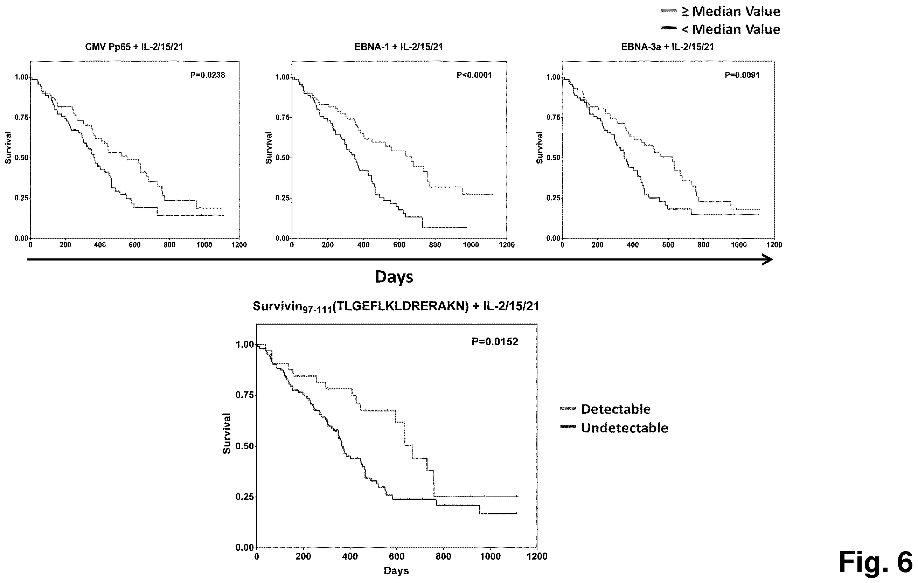

- Fig. 6: **shows antigen-specific IFN-γ responses and survival of patients with GBM based on univariate analysis**. Diluted peripheral blood of patients with GBM was exposed to viral targets (EBNA-1, EBNA-3a and CMV pp65) as well as TAAs (EGFRvIII survivin and NY-ESO-1 peptide pools, respectively) over a 7-day period. Supernatants were harvested for measuring antigen-specific IFN-γ production. Kaplan-Meier survival curves show the relationship between IFN-γ concentrations and patient survival based on median concentration values (viral targets, since the net IFN-γ was very high after medium control subtraction) or detectable and non-detectable IFN-γ levels (for TAAs, since the net IFN-γ production was generally low after medium control subtraction) after antigen stimulation. Blood samples from 145 patients with GBM were used in all WBA except for the testing of survivin₉₇₋₁₁₁ peptide, for which blood samples from 134 patients with GBM were used. Only antigen-specific IFN-γ responses with a statistically significant positive correlation with patient survival are shown. IFN-γ production in response to NY-ESO-1, survivin as well as EGFRvIII peptide pools, respectively do not have a statistically significant correlation with improving the survival of patients with GBM.

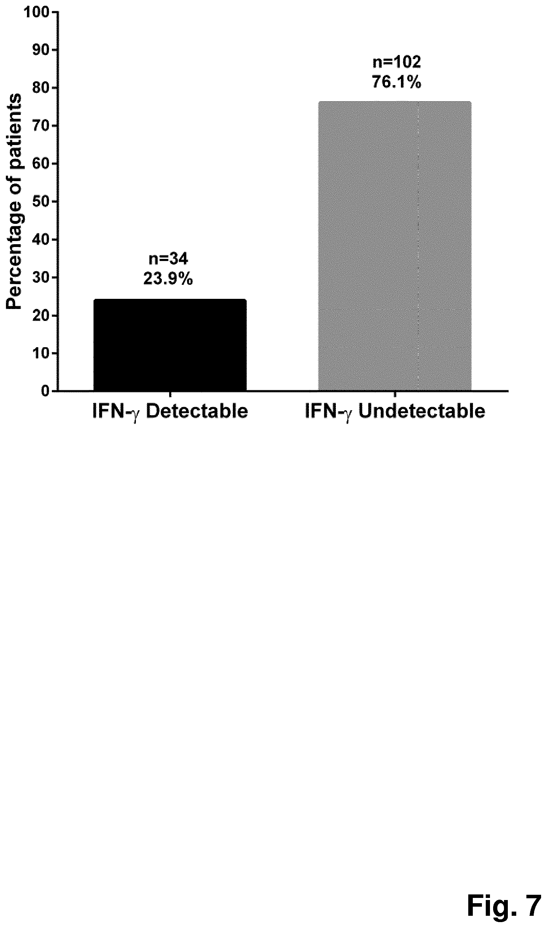

- Fig. 7: **shows the immune response of patients with GBM to survivin**₉₇₋₁₁₁ **peptide (SEQ ID NO:1 TLGEFLKLDRERAKN)**. Specific IFN-γ responses to survivin₉₇₋₁₁₁ peptide were tested with blood samples from 134 patients with GBM using the same WBA method described in this study. 23.9% of patients with GBM (n=32) showed positive reactivity to the survivin₉₇₋₁₁₁ peptide, defined by detectable IFN-γ production in WBA supernatants, while the remaining 76.1% of patients (n=102) did not have a measurable IFN-γ response.

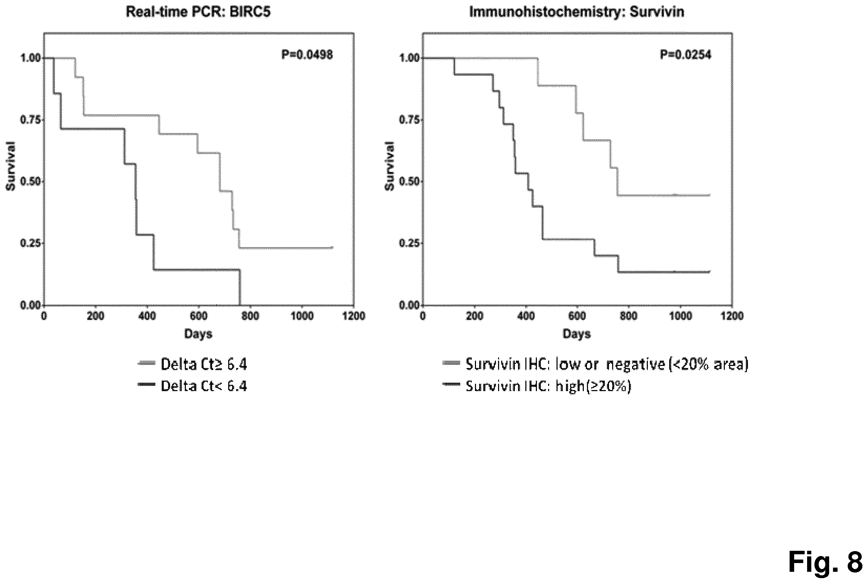

- Fig. 8: **shows the intratumoural survivin/BIRC5 expression in relation to survival of patients with GBM.** Kaplan-Meier survival analyses showing BIRC5 gene expression (A) as well as survivin protein expression (B) in GBM tissue samples in relation to the survival pattern of patients with GBM. Molecular analysis of BIRC5 gene transcription levels in GBM tissue was measured by real-time polymerase chain reaction, while survivin protein expression in paraffinised GBM tissue sections was detected using immunohistochemistry. For BIRC5 gene transcription, low expression indicates a delta cycle threshold (CT) value of >6.4, while high expression indicates a delta CT value of <6.4. Twenty samples from patients with GBM were tested. ΔCT range was 3.56-10.00 and the median ΔCT value was 6.4. Thirteen samples exhibited ΔCT≥6.4 and the remaining seven samples with ΔCT<6.4.

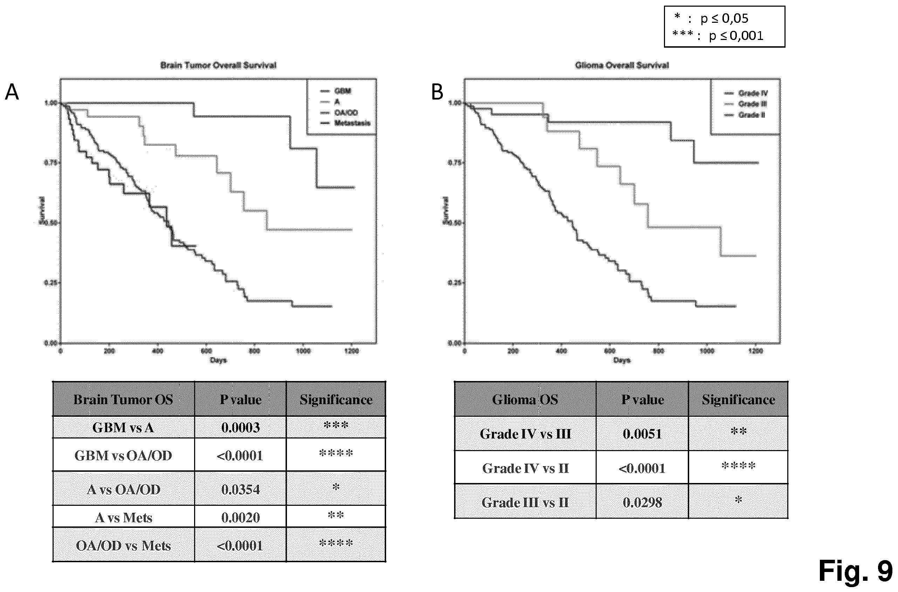

- Fig. 9: **shows the survival pattern of patients with brain tumour in the study cohort.** Patients with different diagnoses of brain tumour were clinically followed up until 1200 days (40 months) post-surgery. Kaplan-Meier survival analyses were performed based on brain tumour type (A) and WHO grading of CNS tumours (B). Legend: A - astrocytoma, OA/OD-oligoastrocytoma/oligodendroglioma, GBM - glioblastoma multiforme. *p<0.05, **p<0.01, **p<0.001, ****p<0.0001.

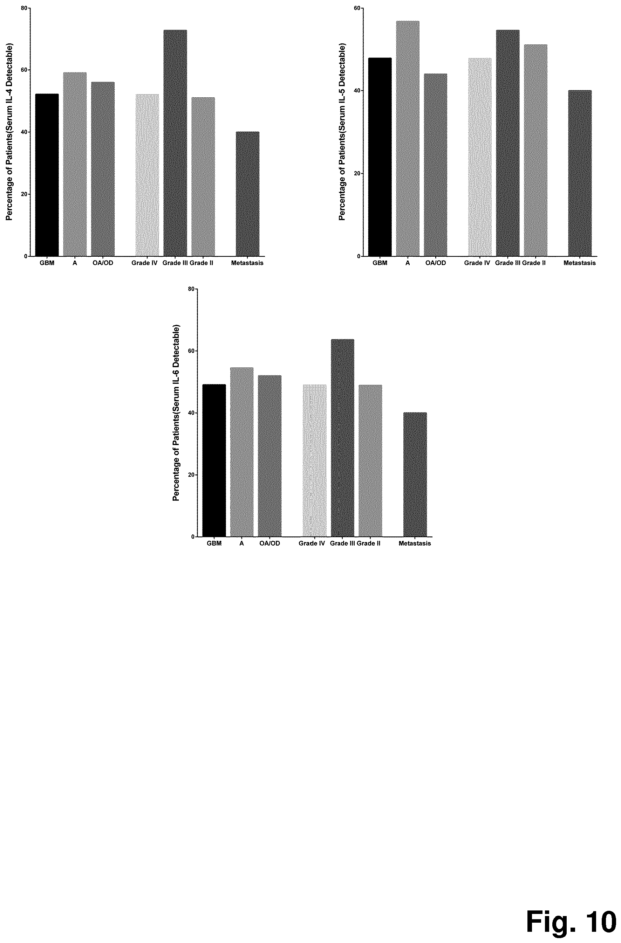

- Fig. 10: **shows anti-inflammatory/pleiotropic cytokine levels in serum of patients with brain tumour.** Sandwich ELISA was used for measuring levels of IL-4, IL-5 and IL-6 in the sera of patients with GBM and expressed in pg/ml. The patients per glioma type with detectable amounts of cytokine (>0 pg/ml) were presented as a fraction (in percentages) of the total number of individuals in the group.

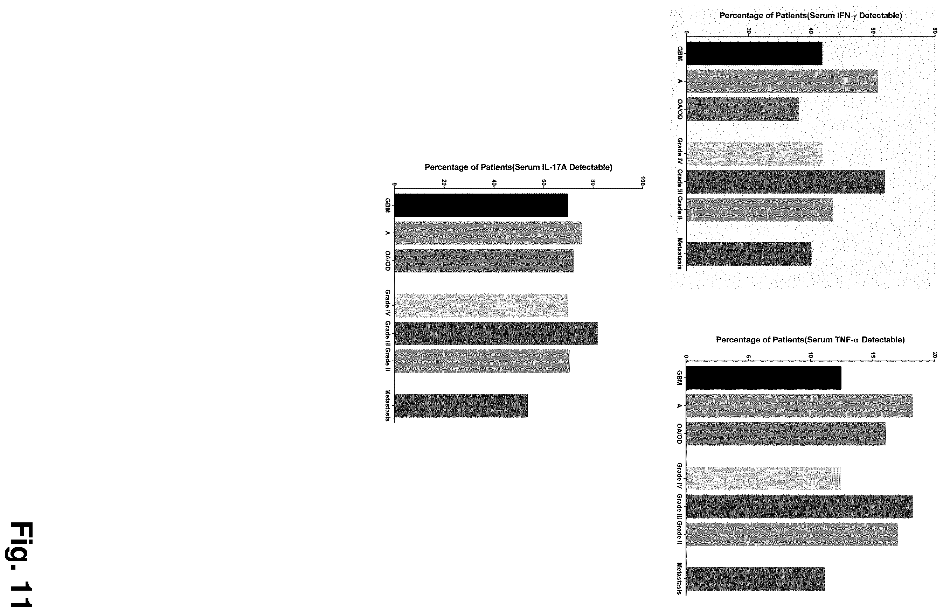

- Fig. 11: **shows pro-inflammatory/Thl cytokine levels in serum of patients with brain tumour.** Sandwich ELISA was used for measuring levels of IFN-γ, TNF-α and IL-17A in the sera of patients with GBM and expressed in pg/ml. The patients per glioma type with detectable amounts of cytokine (>0 pg/ml) were presented as a fraction (in percentages) of the total number of individuals in the group.

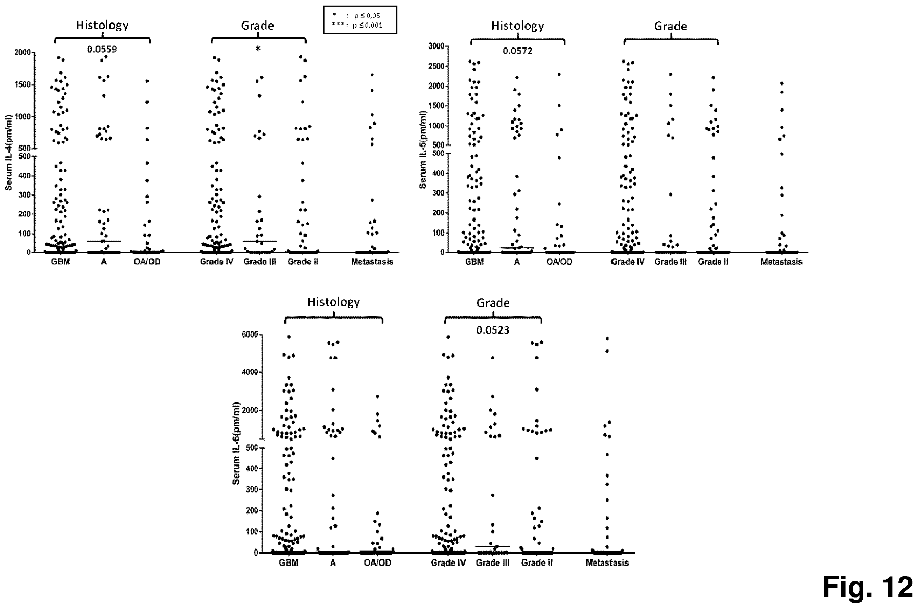

- Fig. 12: **shows IL-4/5/6 levels in brain tumour patients**

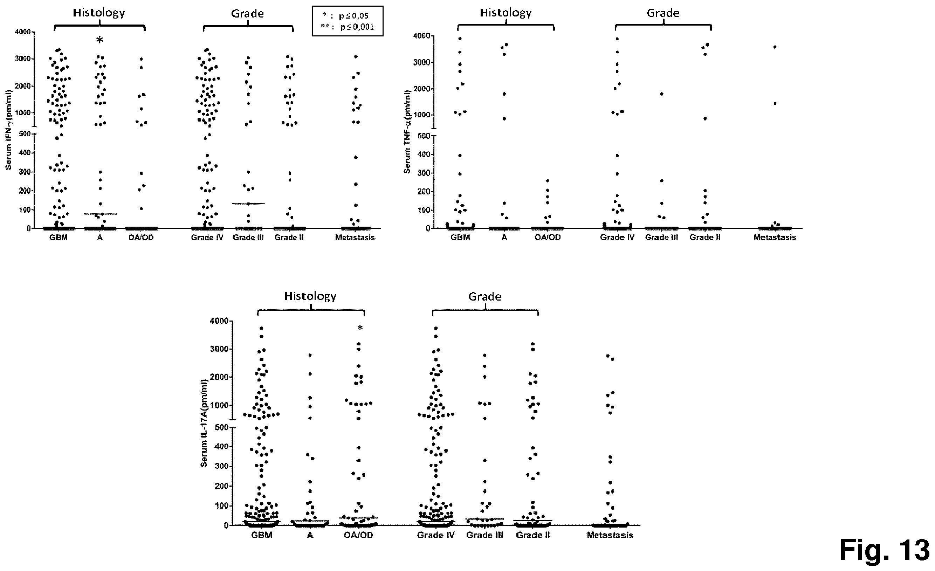

- Fig. 13: **shows IFN-γ/TNF-α/IL-17A levels in brain tumour patients**

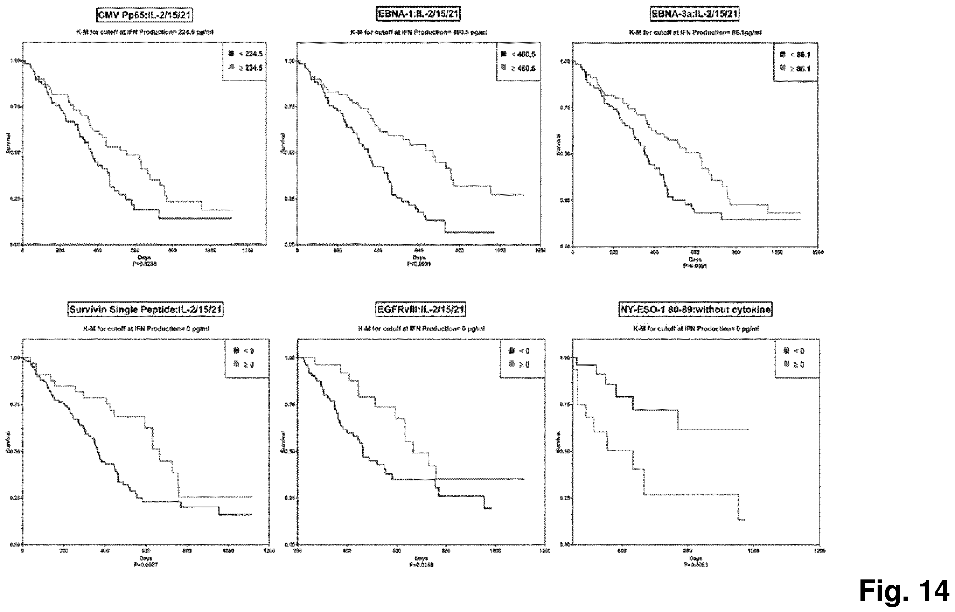

- Fig. 14: **shows antigen-specific IFN-γ responses and survival of patients with GBM.** Diluted peripheral blood of patients with GBM was exposed to EBV antigens (EBNA-1 and EBNA-3a), CMV pp65, EGFRvIII, survivin and NY-ESO-1 over a 7-day period. Supernatants were harvested for measuring antigen-specific IFN-γ production. Kaplan-Meier survival curves show the relationship between IFN-γ concentrations and patient survival based on high and low responders. In general, IFN-γ responses to viral peptide antigens (CMV pp65, EBNA-1, EBNA-3a) were higher than to the tumour peptide antigens (survivin, EGFRvIII and NY-ESO-1).

### DETAILED DESCRIPTION OF THE INVENTION

In order to provide a clear and consistent understanding of the specification and claims, and the scope to be given such terms, the following definitions are provided.

### Definitions

The term "**antibody**" as used herein also includes an "antigen-binding portion" of an antibody (or simply "antibody portion"). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment, which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv). Such single chain antibodies are also intended to be encompassed within the term "antigen-binding portion" of an antibody. Any VH and VL sequences of specific scFv can be linked to human immunoglobulin constant region cDNA or genomic sequences, in order to generate expression vectors encoding complete IgG molecules or other isotypes. VH and VI can also be used in the generation of Fab, Fv or other fragments of immunoglobulins using either protein chemistry or recombinant DNA technology. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites.

An "**antigen**" is any structural substance which serves as a target for the receptors of an adaptive immune response, T cell receptor (TCR) and antibody, respectively. Antigens are, in particular, proteins, polysaccharides, lipids. With respect to proteins being antigens, it is noteworthy that antigenic effect of a protein is the result of one or more epitopes within the amino acid sequence of the protein and that such antigenic epitopes can be preserved in isolated substructures of the protein, namely in protein fragments and individual peptides derived from the protein. Lipids and nucleic acids are particularly antigenic when combined with proteins or polysaccharides. Accordingly, "clinically-relevant antigens" are antigens involved in a disease.

A "**peptide**" as used herein relates to a chain of amino acids connected by peptide bonds. A peptide may be composed of any number of amino acids of any type, preferably naturally occurring amino acids, which, preferably, are linked by peptide bonds. In particular, a peptide comprises at least 3 amino acids, preferably at least 5, at least 7, at least 9, at least 12, or at least 15 amino acids. Furthermore, there is no upper limit for the length of a peptide. However, preferably, a peptide does not exceed a length of 500 amino acids, more preferably it does not exceed a length of 300 amino acids; even more preferably it is not longer than 250 amino acids. Thus, the term "peptide" includes "oligopeptides", which usually refer to peptides with a length of 2 to 20 amino acids and "polypeptides" with at least 60, at least 80, preferably at least 100 amino acids.

The term "**polypeptide**" as used herein refers to a peptide with at least 60 amino acids. The terms "polypeptide" and "protein" are used interchangeably. The polypeptides and proteins as used herein include chemically synthesised proteins as well as naturally synthesised proteins which are encoded by genes. The polypeptides or proteins may be obtained from a natural source, such as human blood or produced in cell culture as recombinant proteins.

The term "protein" as used herein relates to molecules that may contain one or more polypeptide chains. Proteins with one and one polypeptide chain are often expressed from one gene and are only cleaved following translation of the RNA message. Thus, the terms "protein" and "polypeptide" are often used interchangeably. With respect to proteins being antigens, it is noteworthy that the antigenic effect of a protein is the result of one or more epitopes within the amino acid sequence of the protein and that such antigenic epitopes can be preserved in isolated substructures of the protein, namely in protein fragments and individual peptides derived from the protein.

As used herein, the term "**epitope**" relates to the exact binding position, in particular the amino acids, of a protein, that is bound by a binding protein such as an antibody or a TCR. The epitope may be a folding epitope or a sequence epitope. The sequence epitope consists of a specific number of consecutive amino acids in the amino acid sequence of a protein, protein fragment or peptide. Sequence epitopes may have a length in the range from 2 to 10 amino acids, preferably in the range from 3 to 7 amino acids.

"**Tumour-associated antigens**" or "**TAAs**" are antigens that are presented by MHC I or MHC II molecules or non-classical MHC molecules on the surface of tumour cells. As used herein TAA includes "tumour-specific antigens" which are found only on the surface of tumour cells, but not on the surface of normal cells. TAAs also encompass viral antigens associated with tumours. A non-limiting list of antigens considered TAAs in the context of the present disclosure is provided in **Table 3** below.

As used herein, the term "immune cell" includes cells that are of hematopoietic origin and that play a role in the immune response. Immune cells include lymphocytes, such as B cells and T cells; natural killer cells; myeloid cells, such as monocytes, macrophages, eosinophils, mast cells, basophils, and granulocytes.

The term "*clinically-relevant lymphocytes*" refers to lymphocytes that are specific for and interact with clinically-relevant antigens. There are three groups of clinically-relevant lymphocytes, namely tumour-reactive lymphocytes, infectious disease reactive lymphocytes and autoimmune disease reactive lymphocytes. "Clinically-relevant lymphocytes" are also referred to as antigen-edited lymphocytes. The term clinically-relevant is also used for subgroups of lymphocytes. Particularly preferred clinically-relevant lymphocytes are clinically-relevant T cells or antigen-edited T cells.

As used herein, the term "**T cell**" includes cells bearing a T cell receptor (TCR). Preferably, the term "T cell" includes CD4+ T cells and/or CD8+ T cells. The term T cell also includes both T helper 1 type T cells and T helper 2 type T cells. A T cell can be a naïve T cell, i.e., not an activated or memory T cell or an activated or memory T cell. These cells can be distinguished using cell markers known in the art. For example, activated T cells express markers such as CD152 and CD154. Activated T cells also can be characterised by their enhanced ability to produce cytokines, proliferate, or perform certain effector functions.

A "**chimeric antigen receptor (CAR)**" as used herein is an artificially constructed hybrid protein or polypeptide containing the antigen binding domain of an antibody (e.g., single chain variable fragment (scFv)) linked to T cell signalling domains. Characteristics of CARs include their ability to redirect T cell specificity and reactivity toward a selected target in a non-MHC-restricted manner, exploiting the antigen-binding properties of monoclonal antibodies. The non-MHC-restricted antigen recognition gives T cells expressing CARs the ability to recognise antigen independent of antigen processing, thus bypassing a major mechanism of tumour escape. Moreover, when expressed in T cells, CARs advantageously do not dimerise with endogenous T cell receptor (TCR) alpha and beta chains.

In addition to the above definitions, and unless the context clearly requires otherwise, throughout the description and the claims, the words "**comprise**", "**comprising**", and the like are to be construed in an inclusive sense as opposed to an exclusive or exhaustive sense; that is to say, in the sense of "including, but not limited to".

Further, reference throughout this specification to "one **embodiment**", "**some embodiments**" or "**an embodiment**" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives "**first**", "**second**", "**third**", etc., to describe a common object, merely indicate that different instances of like objects are being referred to, and are not intended to imply that the objects so described must be in a given sequence, either temporally, spatially, in ranking, or in any other manner.

### Methods of the invention

Described herein are systems and methods for predicting prolonged survival and/or susceptibility to immunotherapy of individual glioblastoma multiforme (GBM) patients.

In the context of the present invention, it is important to remember that sporadic inflammation of the central nervous system (CNS) has been attributed to GBM tumourigenesis. Inflammation is a dynamic and multifactorial process, with soluble mediators such as cytokines playing a pivotal role in orchestrating the immune responses that sustain health or promote disease. It is generally accepted that in the immunopathogenesis of cancer, cytokine networks are crucial in perpetrating disease initiation and establishment.

In this context the involvement of pro-inflammatory as well as pleiotropic cytokines such as IFN-γ, TNF-α, IL-6, IL-8, and IL-17A in the promotion of GBM was previously reported. Further, it is known that untargeted inflammation causes mutations in genes encoding proteins involved in the receptor tyrosine kinase pathway such as in the genes for the epidermal growth factor receptor (EGFR) and in genes of enzymes mediating DNA repair and ensuring genetic stability. The most frequent EGFR mutation associated with GBM is EGFR variant III (EGFRvIII). In addition, GBM tumours are known to be particularly aggressive due to the immunosuppressive microenvironment they create in the diseased brain. In this microenvironment immunosuppressive regulatory T cells are induced and the expression of the programmed cell death protein 1 (PD-1, which plays an important role in down-regulating the immune system and promoting self tolerance by suppressing T cell inflammatory activity) is increased.

In addition, the production of immune-tolerising cytokines such as transforming growth factor beta (TGF-β), IL-10, IL-4, IL-13 and IL-13 has been reported to play an important role in balancing defensive and conducive tumour-induced immune responses. Accordingly, and without wanting to be bound by theory, the immunosuppressive microenvironment generated by GBM leads to a detrimental skewing of the intricate cytokine balance towards a tumour-conducive profile.

Accordingly, in the GBM tumour microenvironment productive immune responses and efficient defence against tumour propagation is significantly impacted leading to the poor prognosis and reduced survival of GBM patients. However, the present inventors have identified specific cytokine combinations as important prognostic indicators/marker both for prolonged survival and increased susceptibility to immunotherapy of a significant number of individual GBM patients.

In particular, the present inventors surprisingly found that an individual GBM patient's cytokine profile in peripheral blood, cerebrospinal fluid (CSF), bone marrow or tumour infiltrating lymphocytes (TIL) obtained prior to tumour surgery could serve as highly reliable predictor for the individual patient's survival following GBM tumour surgery and GBM therapy as well as the patient's susceptibility to immunotherapy.

In one or more preferred embodiments, the present invention relates to a method for the prediction of prolonged survival of a glioblastoma multiforme (GBM) patient, wherein the method comprises determining the cytokine profile in the GBM patient's peripheral blood prior to tumour surgery, wherein the method comprises determining the cytokine profile in a previously-obtained sample of the GBM patient's peripheral blood, cerebrospinal fluid (CSF), bone marrow or tumour infiltrating lymphocytes (TIL) prior to tumour surgery, wherein the identification of all or none of either:
(a) interleukin-4 (IL-4), interleukin-5 (IL-5) and interleukin-6 (IL-6); or
(b) interferon gamma (IFN-γ), tumour necrosis factor alpha (TNF-a) and interleukin-17A (IL-17A),
in the patient's cytokine profile is indicative of the patient's prolonged survival following GBM therapy.

It was particularly surprising that both the presence and absence of all three of either (a) IL-4, IL-5 and IL-6; or (b) IFN-γ, TNF-α and IL-17A are indicative of prolonged survival of GBM patients following GBM therapy.

However, without wanting to be bound by theory, and since the IL-4, IL-5 and IL-6; or IFN-γ, TNF-α and IL-17A levels in serum are intricately linked, the cytokines' effects are also intricately and possibly synergistically linked such that well-orchestrated and tightly-regulated networks of cytokine combinations are required. This is further illustrated by the fact that it is close to impossible to assign only positive or only negative effects to a single cytokine. For example, cytokines such as IL-10 and others have been described as having both positive or negative effects depending on their concentrations/levels. However, in the present case individual levels of each of the three cytokines of both inventive cytokine combinations are not relevant for prolonged survival as the total measures of "presence" or "absence" of the combinations are relevant.

As such, the present invention underlines the above concept that cytokines within cytokine networks influence each other. As a result, the effects mediated by cytokines are also controlled by the intricate balance of cytokines within cytokine combinations within the overall network, i.e. a properly balanced network of cytokines and cytokine combinations could be considered as the desired steady state or "equilibrium" leading to the desired, physiological effects promoting health. However, disturbances of these networks, for example by the partial removal of some but not all cytokines of the particular cytokine combinations of the present invention lead to undesirable imbalances promoting disease rather than health. The fact that the total absence of the particular cytokine combinations of the present invention is still better than the presence of some of its components, i.e. a partial combination, illustrates that a disturbances in crucial cytokine combinations can cause more damage than removing the entire cytokine combination from the network.

Preferably, the patient's survival is prolonged compared to the survival of GBM patients whose peripheral blood, cerebrospinal fluid (CSF), bone marrow or tumour infiltrating lymphocytes (TIL) cytokine profile prior to tumour surgery includes some but not all of: (a) IL-4, IL-5 and IL-6; or (b) IFN-γ, TNF-α and IL-17A. In particular, the patient's survival is prolonged compared to that of GBM patients whose peripheral blood, cerebrospinal fluid (CSF), bone marrow or tumour infiltrating lymphocytes (TIL) cytokine profile prior to tumour surgery includes: (a) IL-4 and IL-5 but not IL-6; or (b) IL-5 but not IL-4 and IL-6.

Typically, the GBM therapy comprises tumour surgery, radiotherapy as well as the administration of a chemotherapeutic agent, which can be administered orally, intravenously, intra-arterially, intrathecally, infusion via cerebrospinal fluid (CSF)-pathways including shunts and reservoirs, or via implantation of a dissolving wafer, preferably at the site of the resected tumour. In preferred embodiments of the invention, the chemotherapeutic agent is selected from the group consisting of: temozolomide (TMZ); 1,3-Bis(2-chloroethyl)-1-nitrosourea (Carmustine); 1-(2-chloroethyl)-3-cyclohexyl-1-nitrosourea (Lomustine); targeted chemotherapeutic agents such as 3-(1-Methylindol-3-yl)-4-[1-[1-(pyridin-2-ylmethyl)piperidin-4-yl]indol-3-yl]pyrrole-2,5-dione (enzastaurin); angiogenesis inhibitors such as 2-[(2S,5R,8S,11S)-5-benzyl-11-(3-[(diaminomethylidene)amino]propyl}-7-methyl-3,6,9,12,15-pentaoxo-8-(propan-2-yl)-1,4,7,10,13-pentaazacyclopentadecan-2-yl]acetic acid (cilengitide); and angiogenesis/mTOR inhibitors such as (1R,2R,4S)-4-{(2R)-2-[(3S,6R,7E,9R,10R,12R,14S,15E,17E,19E,21S,23S,26R,27R,34aS)-9,27-dihydroxy-10,21-dimethoxy-6,8,12,14,20,26-hexamethyl-1,5,11,28,29-pentaoxo-1,4,5,6,9,10,11,12,13,14,21,22,23,24,25,26,27,28,29,31,32,33,34,34a-tetracosahydro-3H-23,27-epoxypyrido[2,1-c][1,4]oxazacyclohentriacontin-3-yl]propyl}-2-methoxycyclohexyl 3-hydroxy-2-(hydroxymethyl)-2-methylpropanoate (temsirolimus) or dihydroxy-12-[(2R)-1-[(1S,3R,4R)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0 hexatriaconta-16,24,26,28-tetraene-2,3,10,14,20-pentone (everolimus).

In addition to the three fundamental pillars of GBM therapy, namely tumour surgery, radiotherapy and chemotherapy, GBM therapy in accordance with the present invention can further comprise adjunctive immunotherapy. Such adjunctive immunotherapy comprises the administration of an additional immunotherapeutic agent. Typically the immunotherapeutic agent used in adjunctive GBM immunotherapy is selected from the group consisting of: an anti-vascular endothelial growth factor (VEGF) antibody, preferably bevacizumab; programmed death-ligand inhibitors (PD-/PD-L1) or epidermal growth factor inhibitors, preferably gefitinib or erlotinib; and tyrosine kinase receptor inhibitors targeting epidermal growth factor receptors.

In fact, in one or more further preferred embodiments, the present invention relates to a method of predicting a GBM patient's increased susceptibility to immunotherapy. This particular method also comprises determining the cytokine profile in a previously-obtained sample of the GBM patient's peripheral blood, cerebrospinal fluid (CSF), bone marrow or tumour infiltrating lymphocytes (TIL) prior to tumour surgery, wherein the identification of all or none of either:
(a) interleukin-4 (IL-4), interleukin-5 (IL-5) and interleukin-6 (IL-6); or
(b) interferon gamma (IFN-γ), tumour necrosis factor alpha (TNF-a) and interleukin-17A (IL-17A),
in the patient's cytokine profile is indicative of the patient's increased susceptibility to immunotherapy.

As previously indicated, individual cytokines and even cytokine levels are orchestrated in intricate networks in a healthy physiological context to promote health but disturbances in these networks and in the particular cytokine combinations of the present invention lead to a scenario in which disease, and in particular GBM, is promoted. Accordingly, and as described with respect to the other aspect of the present invention, the particular cytokine combinations of the present invention can serve as predictors of prolonged survival. However, similarly, the cytokine pattern with respect to these combinations also provides an indication of a GBM patient's susceptibility to immunotherapy aimed at restoring the balance, for example by completing a partial cytokine combination through the administration of the one or two missing cytokines (cytokine supplementation) or, alternatively, by removing the one or two cytokines present (cytokine depletion).

Administration of immunotherapy compositions, such as cytokine supplementation or cytokine depletion compositions to GBM patients aimed at either completing or removing the particular cytokine combinations of the present invention in their entirety is likely to: i) stop disease progression; ii) lead to tumour regression; iii) lead to stable disease; iv) contain local disease; v) aid to establish long term memory immune responses; vi) facilitate expansion of clinically and biologically relevant immune cell that are able to kill and contain tumour cells; and/or vii) impact on mutational load mediated by inflammation.

Therefore, generally, the patient's susceptibility to immunotherapy is increased compared to that of GBM patients whose peripheral blood, cerebrospinal fluid (CSF), bone marrow or tumour infiltrating lymphocytes (TIL) cytokine profile prior to tumour surgery includes: (a) IL-4 and IL-5 but not IL-6; or (b) IL-5 but not IL-4 and IL-6 and the immunotherapy is adjunctive to GBM therapy. In particular, the immunotherapy comprises the administration of an immunotherapeutic agent selected from the group consisting of: an anti-vascular endothelial growth factor (VEGF) antibody, preferably bevacizumab; programmed death-ligand inhibitors (PD-/PD-L1) or epidermal growth factor inhibitors, preferably gefitinib or erlotinib; and tyrosine kinase receptor inhibitors targeting epidermal growth factor receptors
Preferably, in the above-described methods, the patient sample is a previously-obtained whole blood sample of the patient's peripheral blood.

As will be outlined again in the experimental Examples below, the present inventors have shown for the first time that a combination of cytokines detected in a GBM patient's peripheral blood, cerebrospinal fluid (CSF), bone marrow or tumour infiltrating lymphocytes (TIL), namely the combinations of (a) IL-4, IL-5 and IL-6; or (b) IFN-γ, TNF-α and IL-17A, can reliably predict the individual patient's survival following surgery and GBM therapy.

### Examples

The invention is further described by the following non-limiting Examples.

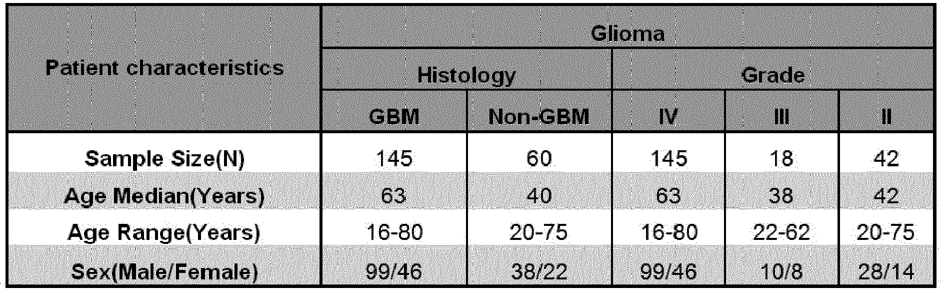

### Patient cohort

The clinical study underlying the present invention and to which the below Examples relate was approved by the Regional Ethics Review Board (Regionala etikprövningsnämnden) at Karolinska Institutet, Stockholm (ethical permit number: 2013/576-31). 206 Patients were selected to participate in the study, after obtaining written informed consent: glioblastoma (GBM, WHO grade IV CNS tumour, n=145) or non-GBM (n=60), comprising patients with astrocytoma, oligodendroglioma as well as oligoastrocytoma or anaplastic oligoastrocytoma (WHO grades II-III CNS tumours). Venous blood for laboratory studies was drawn from the participating patients on the day of the surgery and prior to initiation of chemotherapy. A description of the patient cohort is provided in **Table 1**.

*Table 1: Summary of the clinical characteristics of patients with glioma participating in this study. GBM: Glioblastoma multiforme.*

### Whole blood assay (WBA)

Venous peripheral blood from the patients with glioma was first diluted at a ratio of 1:1.5 with RPMI 1640 Glutamax medium (ThermoFisher Scientific, Carlsbad, CA) and supplemented with antibiotics (penicillin, 100IU/ml and streptomycin, 100µg/ml; ThermoFisher Scientific, Carlsbad, CA). The diluted blood was then conditioned in following manner: i) without cytokines (RPMI only); ii) human IL-7 (10ng/ml) and IL-2 (500IU/ml) or iii) human IL-2 (1000IU/ml), IL-15 (10ng/ml) and IL-21 (10ng/ml; Prospec, Ness-Ziona, Israel) and added to 96-well microtitre plates containing a panel of tumour-associated antigens (TAA) and viral antigens (**Table 2**).

The plates were incubated at 37°C with 5% CO2 for seven days. Incubation with medium alone was used as negative control while 5µg/ml phytohaemagglutinin (PHA, Sigma-Aldrich, St. Louis, MO), 30ng/ml OKT3 (anti-human CD3 monoclonal antibody, Biolegend, CA) and 10 ng/ml SEA+SEB (Staphylococcal Enterotoxin A and B, Sigma-Aldrich, St. Louis, MO) were used as positive controls.

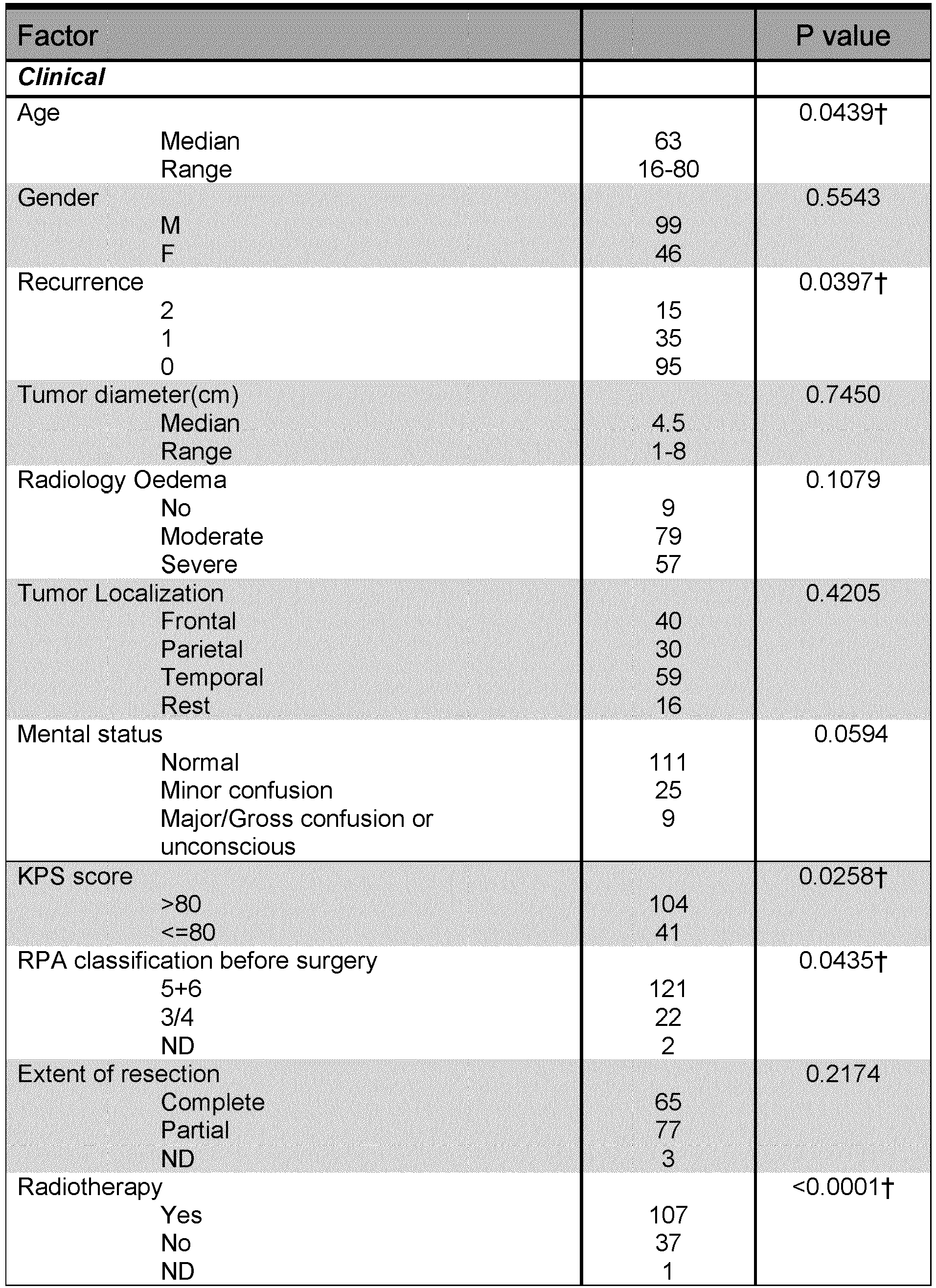

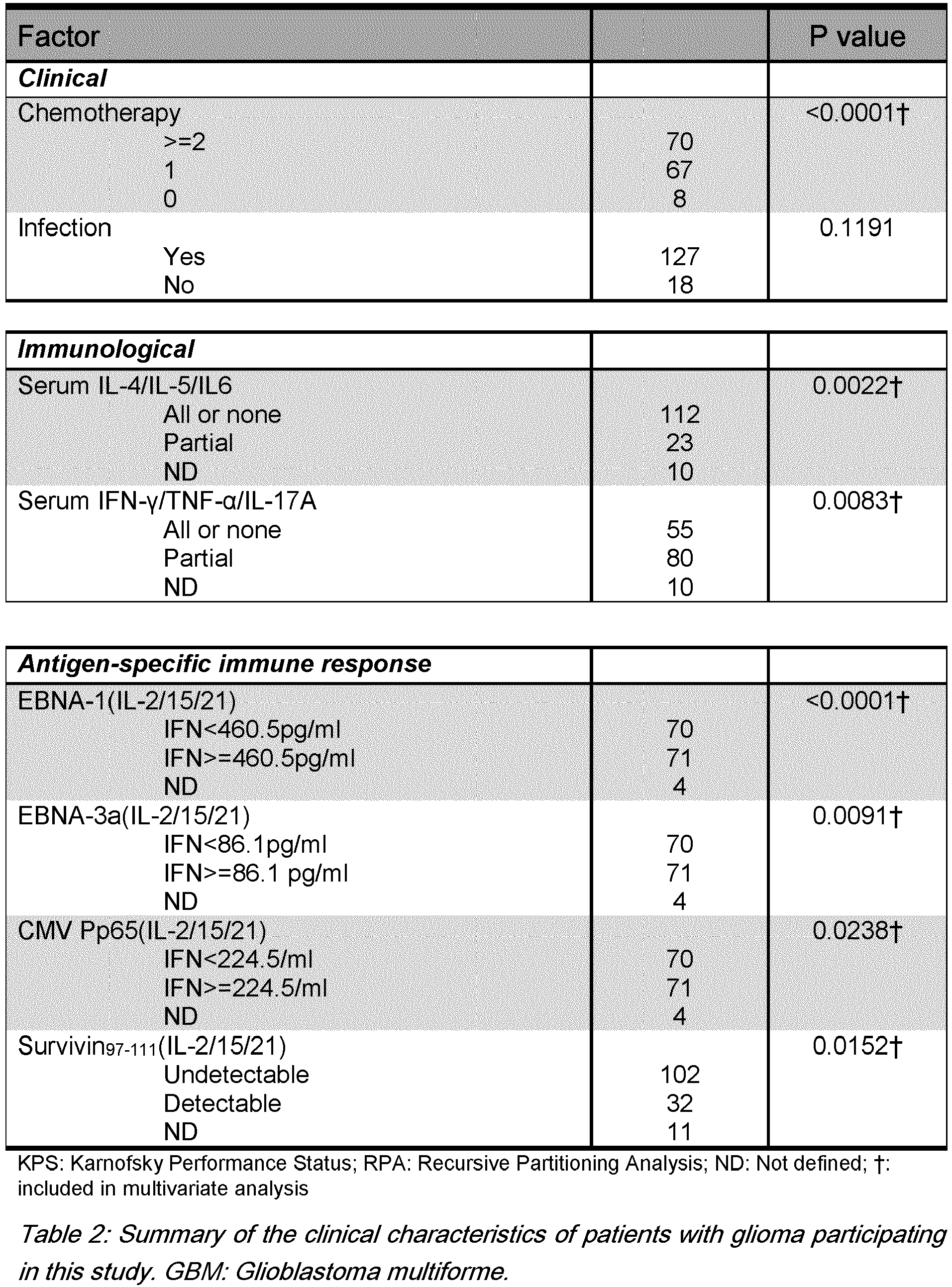

### Blood serum preparation and cytokine ELISA

After drawing venous blood from the participating patients, a fraction of the sample was used for serum preparation. Blood was layered onto Ficoll-Paque Plus solution (GE Healthcare, Uppsala, Sweden), and centrifuged at 1260xg for 10 min. The resulting layer of serum was removed and stored at -80°C. The quantification of cytokines (IFN-γ, TNF-α, IL-17A, IL-4, IL-5 and IL-6) in serum as well as whole blood assay supernatants was performed with commercially available sandwich enzyme-linked immunosorbent assay (ELISA) kits (MABTECH, Stockholm, Sweden) according to the manufacturer's instructions.

### immunohistochemistry

Immunostaining for survivin was performed on 4µm sections of formalin fixed paraffin-embedded tissue using the Leica Bond-Max automated immunostaining system (Leica Biosystems AB, Kista, Sweden). For antigen retrieval, samples were incubated for 20 min at 100 °C with Bond Epitope Retrieval Solution 1 (Leica Biosystems AB, Kista, Sweden). Slides were stained for 30 min at room temperature with the survivin polyclonal antibody RB-9245 (Thermo scientific, Carlsbad, CA), diluted 1:200. The percentage of positive cells was evaluated using a semiquantitative score: 1+ <10%, 2+ = 10-20%, 3+ = 20-50% and 4+ >50% (the high-expression group refers to score 4 and 3, while the 'low-expression' group includes score 2 and 1.

### Real-time polymerase chain reaction (RT-PCR)

Qualitative BIRC5 PCR: Total RNA was extracted from flash frozen tumour specimens obtained during surgery. The tumour tissue was lysed using Qiazol tissue lysis reagent (Qiagen Inc., Hilden, Germany) and total RNA was obtained by ethanol precipitation according to supplier's instructions. 5 µg total RNA was subjected to reverse transcription using an Oligo DT protocol of a RevertAid first strand cDNA synthesis kit (Thermo Fisher Scientific inc. Waltham, MA, USA) according to the manufacturer's instructions. 10µl cDNA from each cDNA reaction was used to amplify the BIRC5 variant specific product in a 20 µl PCR reaction using BIRC5 commercially available transcript specific primers published previously and according to the manufacturer's instructions (ThermoFisher Scientific, Carlsbad, CA). Examplary primers for BIRC5/surviving are:
**survivin-S:** 20-mer, 5' to 3', SEQ ID NO:2: ATG GGT GCC CCGACG TTG CC; and
**survivin-A:** 20-mer, 5' to 3', SEQ ID NO:3: GCA GCT CCG GCC AGA GGC CT).

β-actin was used as positive control for housekeeping gene transcription. The PCR reaction was initiated at 95°C for 10 minutes followed by 40 cycles at 95°C for 15 seconds, 60°C for 30 seconds and 72°C for 30 seconds. ΔCt for each sample refers to the difference between Ct values of BIRC5 and β-actin. Cut-off for values of Ct and ΔCt is low than 35 and 10 respectively.

### Statistical methods, survival curves, patient stratification

The Kaplan-Meier (K-M) survival analysis was used for estimation of the number of patients who survived over the follow-up period of 1200 days. Univariate analysis was performed by comparing single parameters (clinical, immunological and antigen-specific immune response) with the overall survival of patients with GBM Cox Proportional hazards model (forward and backward stepwise analysis) for multivariate analysis was applied to account for individual parameters/factors that could predict improved survival of patients when analysed in combination with all the parameters considered in the study. For the relationship between IFN-γ production by peripheral blood and patient survival, cut-offs for K-M analysis were decided based on 'detectable' or 'non-detectable' levels (assay detection limit provided by the manufacturer) or the median concentration values of cytokines in samples which could generate the greatest hazard ratios between two groups.

### Example 1: Clinical characteristics and survival pattern of patients with malignant glioma

Patients with malignant glioma (n=205) enrolled in this study (**Table 1**) were categorised into two distinct groups: (i) patients with GBM and (ii) patients with non-GBM malignant glioma (astrocytoma, oligodendroglioma or oligoastrocytoma with different subtypes). The median age for patients with GBM (n=145) was 63 years, while patients in the non-GBM group (n=60) had a median age of 40 years. There were 137 male patients and 68 female patients in the study cohort. Approximately 18% of patients with non-GBM malignant glioma were still alive at 1000 days post-surgery, as opposed to a meagre 2% of patients with GBM (**Figure 1**). Thus, patients with GBM exhibited the weakest survival pattern post-surgery, compared to individuals with non-GBM malignant glioma (p<0.0001; **Figure 1**; see also **Figure 9**).

### Example 2: Clinical characteristics and survival pattern of patients with malignant glioma

After drawing venous blood from the participating patients, a fraction of the sample was used for serum preparation. Blood was layered onto Ficoll-Paque Plus solution (GE Healthcare, Uppsala, Sweden), and centrifuged at 1260xg for 10 min. The resulting layer of serum was removed and stored at -80°C. The quantification of cytokines (IFN-γ, TNF-α, IL-17A, IL-4, IL-5 and IL-6) in serum as well as whole blood assay supernatants was performed with commercially available sandwich enzyme-linked immunosorbent assay (ELISA) kits (MABTECH, Stockholm, Sweden) according to the manufacturer's instructions.

Serum cytokines were separated into two functional groups: (i) Th2/anti-inflammatory and (ii) Th1/inflammatory mediators. IL-6 was included in the first group, since it has both pro-and anti-inflammatory properties (pleiotropic soluble mediator). Approximately 50% of patients with GBM (identical to WHO grade IV CNS tumour) exhibited detectable levels of IL-4, IL-5 and IL-6 in serum; while up to 60% of patients with non-GBM diagnoses of glioma had detectable levels of all three cytokines (**Figure 2A**). Based on the WHO grading of CNS tumours, between 53% and 60% of patients with grade II/III glioma had detectable levels of IL-4, IL-5 and IL-6 (**Figure 2A**).

A smaller fraction of patients with GBM, compared to patients with non-GBM malignant gliomas, had detectable levels of serum IFN-γ (41% vs. 52%) and TNF-α (12% vs. 19%; **Figure 2B**). The percentage of patients with detectable levels of IL-17A in serum was similar among the GBM, and non-GBM malignant glioma categories. Higher levels of IFN-γ (52%), TNF-α (19%) and IL-17A (78%) were also detected in the sera of patients with WHO grade II/III compared to patients with grade IV glioma (GBM; **Figure 2B**). The inventors also observed that the median values of cytokine concentrations in the serum samples did not differ between patients with GBM and patients with non-GBM malignant glioma (**Figure 3**; see also **Figures 10** and **11**).

### Example 3: Combinatorial effect of serum cytokines on patient survival patterns

Using a multivariate analysis model to visualise the effect of serum cytokines on the survival of patients with GBM, the inventors found that either the entire set of IL-4, IL-5 and IL-6 as a pattern, i.e. either three cytokines all present or all absent ('all' or 'none') correlated with a better survival profile(p=0.0022) among the patients compared to only a 'partial' combination(e.g. IL-4 and IL-5 are detectable but no IL-6, OR il-5 is detectable but no IL-4 and IL-6). (**Figure 5A**). The scenario is similar for IFN-γ/TNF-α/IL-17A levels in serum; patients with all of the cytokines or none of them tend to exhibit an improved survival pattern (p=0.0083; **Figure 5B**). The inventors also noticed that an existing interaction between detectable serum levels of IL-4 and IL-5 (R²=0.7832, p<0.05), IL-4 and IL-6 (R²=0.8104, p<0.05) and IL-5 and IL-6 (R²=0.8160, p<0.05) and improved patient survival. The same was true for the interaction between detectable serum levels of IFN-γ and IL-17A (R²=0.9161, p<0.05). Also refer to **Figures 12** and **13**.

### Example 4: Antigen-specific IFN-γproduction and association with survival pattern of patients with GBM

Venous peripheral blood from the patients with glioma was first diluted at a ratio of 1:1.5 with RPMI 1640 Glutamax medium (ThermoFisher Scientific, Carlsbad, CA) and supplemented with antibiotics (penicillin, 100IU/ml and streptomycin, 100µg/ml; ThermoFisher Scientific, Carlsbad, CA). The diluted blood was then conditioned in following manner: i) without cytokines (RPMI only); ii) human IL-7 (10ng/ml) and IL-2 (500IU/ml) or iii) human IL-2 (1000IU/ml), IL-15 (10ng/ml) and IL-21 (10ng/ml; Prospec, Ness-Ziona, Israel) and added to 96-well microtitre plates containing a panel of tumour-associated antigens (TAA) and viral antigens (**Table 3**). The plates were incubated at 37°C with 5% CO₂ for seven days. Incubation with medium alone was used as negative control while 5µg/ml phytohaemagglutinin (PHA, Sigma-Aldrich, St. Louis, MO), 30ng/ml OKT3 (anti-human CD3 monoclonal antibody, Biolegend, CA) and 10 ng/ml SEA+SEB (Staphylococcal Enterotoxin A and B, Sigma-Aldrich, St. Louis, MO) were used as positive controls.

**Table 3: List of antigens used in the Whole Blood Assays (WBA) in this study.**

| **Antigen** | **Type** | **Final concentration** | **Access NO.** |
|---|---|---|---|
| PHA | protein | 5µg/ml | L901710MG |
| CD3 Ab | protein | 30ng/ml | Clone:OKT3 |
| SEA+SEB | protein | 10ng/ml | SEA:Sigma#9399SEB:S-4481 |
| EBNA-1 | protein | 1µg/ml | P03211 (Uniprot) |
| EBNA-3a | protein | 1 µg/ml | P12977(Uniprot) |
| A-VIET(H5N1) | protein | 2.5µg/ml | Baxter (HA protein) |
| H1N1 California | protein | 2.5µg/ml | GSKsplit |
| ESAT-6 | protein | 1 µg/ml | AEP68523 |
| CMV-pp65 | protein | 1 µg/ml | P06725 (Uniprot) |
| EGFRvIII | peptide | 1 µg/ml | LEEKKGNYWTDH (SEQ ID NO:5) |
| NY-ESO-1 | peptide mix | 1 /peptide/ml | P78358 (Uniprot) |
| NY-ESO-1 80-94 | peptide | 1 µg/ml | ARGPESRLLEFYLAM (SEQ ID NO:4) |
| NY-ESO-1 89-103 | peptide | 1 µg/ml) | EFYLAMPFATPMEAE (SEQ ID NO:6) |
| NY-ESO-1 157-171 | peptide | 1 µg/ml | SLLMWITQCFLPVFL (SEQ ID NO:7) |
| survivin single peptide | peptide | 1 µg/ml | TLGEFLKLDRERAKN (SEQ ID NO:1) |
| survivin peptide mix | peptide mix | 1 1/peptide/ml | 015392 (Uniprot) |

IFN-γ responses of peripheral blood mononuclear cells (PBMCs) from patients with GBM were analysed after stimulation with the antigens listed in **Table 3**, with or without cytokine cocktail conditioning. CMV pp65-specific IFN-γ production by PBMCs correlated closely with a better survival profile of the patients in the presence of IL-2, IL-15 and IL-21 conditioning (**Figure 6**). This was also found to be true for IFN-γ production in response to EBNA-1, EBNA-3a or the survivin 97-111 single peptide (SEQ ID NO:1 TLGEFLKLDRERAKN). Conversely, IFN-γ production in response to the NY-ESO1₈₀₋₉₄ single peptide (SEQ ID NO:4 ARGPESRLLEFYLAM) or stimulation with the EGFRvIII₁₋₁₃ peptide (SEQ ID NO:5 LEEKKGNYVVTDH) did not correlate with improved survival of patients with GBM. Also see **Figure 14**.

### Example 5: Univariate and multivariate analyses of immunological and clinical parameters identify targeted IFN-γ responses to survivin₉₇₋₁₁₁ peptide (SEQ ID NO:1 TLGEFLKLDRERAKN) to be associated with improved survival of patients with GBM

All clinical and immunological parameters, as well as the antigen-specific immune responses considered in this study were included in the univariate analysis (**Table 2**). The immunological factors are distinguished from the antigen-specific immune responses, i.e. the former is a measure of circulating cytokines in blood without manipulation of the clinical samples, while the antigen-specific immune responses were measured after *in vitro* manipulation of peripheral blood with/without cytokine conditioning and antigenic stimulation. Each individual clinical, immunological and antigen-specific immune response parameter/factor was tested in relation to patient survival for determining whether they had a role in improving the survival pattern of patients with GBM. The following clinical parameters were found to be strongly associated with improved survival: age of patients (p=0.0439), tumour recurrence (p=0.0397), Karnofsky Performance Status (KPS) of patients (p=0.0258), recursive-partitioning analysis (RPA) before surgery (p=0.0435), radiotherapy (p<0.0001) and chemotherapy (p<0.0001). Both immunological factors were associated with improved survival of patients with GBM: serum levels of IL-4/IL-5/IL-6 (p=0.0022) as well as IFN-γ/TNF-α/IL-17A (p=0.0083). Antigen-specific immune responses (IFN-γ production) to EBNA-1 (p<0.0001), EBNA-3a (p=0.0091), CMV pp65 (p=0.0238) as well as the survivin₉₇₋₁₁₁ peptide (SEQ ID NO:1 TLGEFLKLDRERAKN; p=0.0152) in the WBA with IL-2/IL-15/IL-21 conditioning also correlated with improved survival (**Table 2**). Since none of the antigen-specific immune response parameters in the unconditioned and IL-2/IL-7-conditioned groups gave a *p* value of less than 0.05, and their results are not reported here.

Next, the inventors determined whether the antigen-specific immune responses, together with the clinical and immunological factors measured in this study were capable of predicting improved survival of patients with GBM. All parameters that gave a *ρ* value of less than 0.05 in the univariate analysis from the three separate culture conditions (untreated, IL-2/IL-7 and IL-2/IL-15/IL-21) were fitted into the forward-backward stepwise Cox regression model for multivariate analysis. Parameters that resulted in a significant *p* value (<0.05) in a relation to improved survival of patients with GBM are listed in **Table 4**, illustrating the very essence of the present invention.

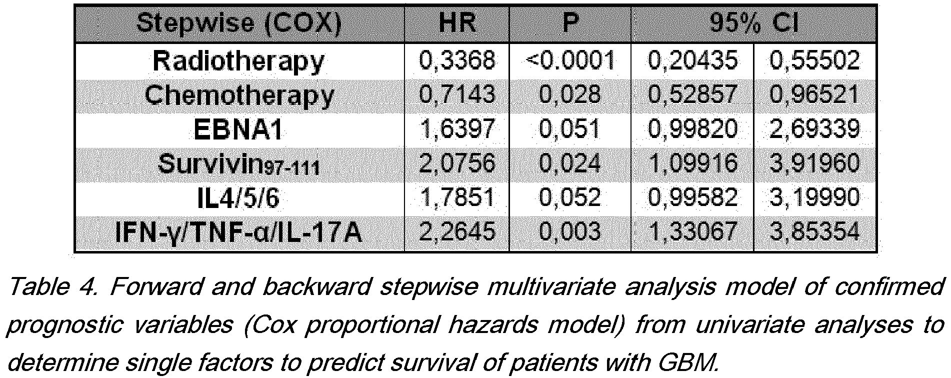

Among all the clinical parameters tested, only radiotherapy (p<0.0001; HR=0.3368) and chemotherapy (p=0.028; HR=0.7143) emerged as clinical parameters that positively correlated with improved survival of patients with GBM since the analysis revealed with high certainty that the risk of death for patients receiving radiotherapy was 0.3368 times of that of patients not receiving radiotherapy (p<0.0001; HR=0.3368) and the risk of death for patients receiving chemotherapy was 0.7143 times of that of patients not receiving chemotherapy (p=0.028; HR=0.7143).

IFN-γ response to the survivin₉₇₋₁₁₁ peptide (SEQ ID NO:1) and EBNA-1 were identified as being associated with improved survival of GBM patients. In accordance with the methods described above, approximately 24% of patients with GBM exhibit IFN-γ responses to the survivin₉₇₋₁₁₁ peptide (SEQ ID NO:1) in their peripheral blood conditioned with IL-2/IL-15/IL-21 (**Figure 7**). As such, the survivin₉₇₋₁₁₁ peptide (SEQ ID NO:1) was identified as the only, single antigen eliciting an antigen-specific immune response that was positively correlated with improved survival of patients, while none of the other single antigen peptides listed in **Table 3** above elicit such responses (also see **Figure 6** and **Figures 12** to **14**).

Specifically, the risk of death for patients whose peripheral blood samples show no or only a weak reaction to EBNA-1, i.e. a response below the median value, is 1.6397 times higher than for patients with strong immune responses to EBNA-1, i.e. a response equal to or above the median value, (p=0.051; HR=1.6397). For patients whose peripheral blood samples does not show a detectable immune response to the survivin₉₇₋₁₁₁ peptide (SEQ ID NO:1), the risk of death is 2.0756 times higher than for patients with a detectable immune response to the survivin₉₇₋₁₁₁ peptide (p=0.024; HR=2.0756).

Multivariate analysis of the immunological parameters of Table 2 confirmed the before-mentioned correlation between the all or none of either:
(a) interleukin-4 (IL-4), interleukin-5 (IL-5) and interleukin-6 (IL-6); or
(b) interferon gamma (IFN-γ), tumour necrosis factor alpha (TNF-a) and interleukin-17A (IL-17A),
with the patient's prolonged survival following GBM therapy.

Specifically, patients with only one or two of IL-4/IL-5/IL-6 had a risk of death that was 1.7851 times higher than that of patients with all or none of IL-4/IL-5/IL-6 (p=0.052; HR=1.7851). Patients with only one or two of IFN-γ/TNF-α/ IL-17 had a risk of death that was 2.2645 times higher than that of patients with all or none of IL-4/IL-5/IL-6 (p=0.003; HR=2.2645).

### Discussion of results obtained in the Examples above

As already indicated above, inflammation is a dynamic and multifactorial process, with soluble mediators such as cytokines playing a pivotal role in orchestrating the immune responses that sustain health or promote disease. It is generally accepted that in the immunopathogenesis of cancer, cytokine networks are crucial in perpetrating disease initiation and establishment. The present studies provide evidence, for the first time, that a combination of cytokines detectable in serum of patients with GBM prior to surgery, namely IL-4, IL-5 and IL-6 or IFN-γ, TNF-α and IL-17A can predict survival profile after surgery and standard therapy.

It is also shown that circulating T cells specific for Eppstein-Barr virus (EBV), cytomegalo virus (CMV), or survivin in patients with GBM can be used to measure clinically-relevant immune responses, which strongly correlate with patient survival. This is also the first study describing IFN-γ responses to a single survivin peptide as a predictor of prolonged survival among patients with GBM.

The relationship between serum cytokines and histopathological findings in patients with GBM was previously explored and an association between up-regulation of IL-8 levels and endothelial hyperplasia as well as coagulation necrosis was reported, while VEGF up-regulation was found to be linked with ischemic necrosis. However, a correlation between patient survival and cytokine levels could not be established. While the levels of individual cytokines in sera have been assessed for their influence on survival of patients with advanced cancer, the effect of cytokine networks remained underexplored.

A cytokine-based immune network scheme as shown by the Examples of the present invention provides an much higher resolution of the disease mechanisms likely to be involved in cancer promotion, thus allowing a deeper insight into biological targets for pharmacological intervention.

The here-demonstrated involvement of IL-6 in the survival of patients with GBM is intriguing, particularly in association with the strictly Th2-associated cytokines IL-4 and IL-5. Although with many pro-inflammatory attributes, IL-6 is a pleiotropic cytokine that also exerts anti-inflammatory effects. In combination with IL-5, IL-6 can support the differentiation and maintenance of plasma cells in the host. IL-6 and IL-4 can synergistically reduce the number of Tregs in circulation by promoting FoxP3 down-regulation. Thus, the combinatorial effect of IL-4, IL-5 and IL-6 shown here provides an opportunity to control the tumour burden in patients by sustaining the secretion of TAA-specific antibodies without Treg-associated disruption of this immunological state. Although IL-5 and IL-4 are commonly associated with Th2 immune responses and anti-inflammatory activity, they can nonetheless be involved in inflammatory processes. IL-5 is associated with eosinophil induction in airway hyper-reactivity and asthma, while IL-4 up-regulation is linked to IL-12p40 production by dendritic cells and Th1 cell induction as well as cytotoxic lymphocyte activation. Since a survival benefit was observed also when none of the above-mentioned cytokines were detected in patients' sera, and without wanting to be bound by theory either the absence of systemic hyper-immune activation or the presence of a balanced cytokine network are likely to contribute to clinically durable anti-tumour immune responses.

The above described results also show that targeted immune responses to the survivin₉₇₋₁₁₁ peptide of SEQ ID NO:1 (represented by antigen-specific IFN-γ production) correlates with 70% of survival at 600 days post-surgery as opposed to under 25% among patients who did not have detectable levels of cytokine responses. This is also reflected in the multivariate analysis, where IFN-γ production in response to the survivin₉₇₋₁₁₁ peptide has emerged as an independent predictor of improved survival of patients with GBM.

The present invention shows that the immunological imprint in patients with GBM strongly influences the clinical outcome of GBM immunotherapy. Patients with GBM who exhibit better survival in relation to an existing combination of circulating cytokines are more receptive to immunotherapy.

### SEQUENCE LISTING

SEQ ID NO:1 (survivin₉₇₋₁₁₁ peptide)
   TLGEFLKLDRERAKN
SEQ ID NO:2 (survivin-S real time PCR primer sequence, 20-mer, 5' to 3')
   ATG GGT GCC CCGACG TTG CC
SEQ ID NO:3 (survivin-A real time PCR primer sequence, 20-mer, 5' to 3')
   GCA GCT CCG GCC AGA GGC CT
SEQ ID NO:4 (NY-ESO1₈₀₋₉₄ peptide)
   ARGPESRLLEFYLAM
SEQ ID NO:5 (EGFRvIII₁₋₁₃ peptide)
   LEEKKGNYWTDH
SEQ ID NO:6 (NY-ESO₁₈₉₋₁₀₃ peptide)
   EFYLAMPFATPMEAE
SEQ ID NO:7 (NY-ESO1₁₅₇₋₁₇₁ peptide)
   SLLMWITQCFLPVFL

## Claims

1. A method for the prediction of prolonged survival of a glioblastoma multiforme (GBM) patient, wherein said method comprises determining the cytokine profile in a previously-obtained sample of the GBM patient's peripheral blood, cerebrospinal fluid (CSF), bone marrow or tumour infiltrating lymphocytes (TIL) prior to tumour surgery, wherein the identification of all or none of either:
(a) interleukin-4 (IL-4), interleukin-5 (IL-5) and interleukin-6 (IL-6); or
(b) interferon gamma (IFN-γ), tumour necrosis factor alpha (TNF-a) and interleukin-17A (IL-17A),
in said patient's cytokine profile is indicative of the patient's prolonged survival following GBM therapy.

2. The method according to claim 1, wherein the patient's survival is prolonged compared to the survival of GBM patients whose peripheral blood, cerebrospinal fluid (CSF), bone marrow or tumour infiltrating lymphocytes (TIL) cytokine profile prior to tumour surgery includes some but not all of:
(a) IL-4, IL-5 and IL-6; or
(b) IFN-γ, TNF-α and IL-17A,
optionally, the patient's survival is prolonged compared to that of GBM patients whose peripheral blood, cerebrospinal fluid (CSF), bone marrow or tumour infiltrating lymphocytes (TIL) cytokine profile prior to tumour surgery includes: (a) IL-4 and IL-5 but not IL-6; or (b) IL-5 but not IL-4 and IL-6,
optionally, the GBM therapy comprises tumour surgery, radiotherapy as well as the administration of a chemotherapeutic agent,
- preferably the chemotherapeutic agent is administered orally, intravenously, intra-arterially, intrathecally, infusion via cerebrospinal fluid (CSF)-pathways including shunts and reservoirs, or via implantation of a dissolving wafer,
- preferably the chemotherapeutic agent is selected from the group consisting of: temozolomide (TMZ); 1,3-Bis(2-chloroethyl)-1-nitrosourea (Carmustine); 1 -(2-chloroethyl)-3-cyclohexyl-1 -nitrosourea (Lomustine); targeted chemotherapeutic agents such as 3-(1-Methylindol-3-yl)-4-[1-[1-(pyridin-2-ylmethyl)piperidin-4-yl]indol-3-yl]pyrrole-2,5-dione (enzastaurin); angiogenesis inhibitors such as 2-[(2S,5R,8S,11S)-5-benzyl-11-{3-[(diaminomethylidene)amino]propyl}-7-methyl-3,6,9,12,15-pentaoxo-8-(propan-2-yl)-1,4,7,10,13-pentaazacyclopentadecan-2-yl]acetic acid (cilengitide); and angiogenesis/mTOR inhibitors such as (1R,2R,4S)-4-{(2R)-2-[(3S,6R,7E,9R,10R,12R,14S,15E,17E,19E,21S,23S,26R,27R,34aS)-9,27-dihydroxy-10,21-dimethoxy-6,8,12,14,20,26-hexamethyl-1,5,11,28,29-pentaoxo-1,4,5,6,9,10,11,12,13,14,21,22,23,24,25,26,27,28,29,31,32,33,34,34a-tetracosahydro-3H-23,27-epoxypyrido[2,1-c][1,4]oxazacyclohentriacontin-3-yl]propyl}-2-methoxycyclohexyl 3-hydroxy-2-(hydroxymethyl)-2-methylpropanoate (temsirolimus) or dihydroxy-12-[(2R)-1-[(1S,3R,4R)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0 hexatriaconta-16,24,26,28-tetraene-2,3,10,14,20-pentone (everolimus),
- preferably the GBM therapy further comprises adjunctive immunotherapy comprising the administration of an immunotherapeutic agent selected from the group consisting of: an anti-vascular endothelial growth factor (VEGF) antibody, preferably bevacizumab; programmed death-ligand inhibitors (PD-/PD-L1) or epidermal growth factor inhibitors, preferably gefitinib or erlotinib; and tyrosine kinase receptor inhibitors targeting epidermal growth factor receptors.

3. A method of predicting a GBM patient's increased susceptibility to immunotherapy, wherein said method comprises determining the cytokine profile in a previously-obtained sample of the GBM patient's peripheral blood, cerebrospinal fluid (CSF), bone marrow or tumour infiltrating lymphocytes (TIL) prior to tumour surgery, wherein the identification of all or none of either:
(a) interleukin-4 (IL-4), interleukin-5 (IL-5) and interleukin-6 (IL-6); or
(b) interferon gamma (IFN-γ), tumour necrosis factor alpha (TNF-a) and interleukin-17A (IL-17A),
in said patient's cytokine profile is indicative of the patient's increased susceptibility to immunotherapy.

4. The method of claim 3, wherein the patient's susceptibility to immunotherapy is increased compared to that of GBM patients whose peripheral blood, cerebrospinal fluid (CSF), bone marrow or tumour infiltrating lymphocytes (TIL) cytokine profile prior to tumour surgery includes: (a) IL-4 and IL-5 but not IL-6; or (b) IL-5 but not IL-4 and IL-6, preferably said immunotherapy is adjunctive to GBM therapy and comprises the administration of an immunotherapeutic agent selected from the group consisting of: an anti-vascular endothelial growth factor (VEGF) antibody, preferably bevacizumab; programmed death-ligand inhibitors (PD-/PD-L1) or epidermal growth factor inhibitors, preferably gefitinib or erlotinib; and tyrosine kinase receptor inhibitors targeting epidermal growth factor receptors.

5. The method of any one of claims 1 to 4, wherein the sample is a previously-obtained whole blood sample of said patient's peripheral blood.

## Patentansprüche

1. Ein Verfahren zur Vorhersage des verlängerten Überlebens eines Patienten mit Glioblastoma multiforme (GBM), wobei das Verfahren das Bestimmen des Zytokinprofils in einer zuvor erhaltenen Probe des peripheren Blutes, der Liquor cerebrospinalis (CSF), des Knochenmarks oder der Tumorinfiltrierenden Lymphozyten (TIL) des GBM-Patienten vor der Tumoroperation umfasst, wobei die Identifizierung jedes oder keines von entweder:
(a) Interleukin-4 (IL-4), Interleukin-5 (IL-5) und Interleukin-6 (IL-6); oder
(b) Interferon Gamma (IFN-γ), Tumornekrosefaktor Alpha (TNF-a) und Interleukin-17A (IL-17A),
im Zytokinprofil des Patienten auf das verlängerte Überleben des Patienten nach einer GBM-Therapie hinweist.

2. Verfahren nach Anspruch 1, wobei das Überleben des Patienten im Vergleich zum Überleben von GBM-Patienten verlängert ist, deren Zytokinprofil des peripheren Blutes, Liquor cerebrospinalis (CSF), Knochenmarks oder der Tumorinfiltrierende Lymphozyten (TIL) vor der Tumoroperation einige aber nicht alle von:
(a) IL-4, IL-5 und IL-6; oder
(b) IFN-γ, TNF-α und IL-17A umfasst;
optional ist das Überleben des Patienten im Vergleich zu GBM-Patienten verlängert, deren Zytokinprofil des peripheren Blutes, Liquor cerebrospinalis (CSF), Knochenmarks oder der Tumorinfiltrierenden Lymphozyten (TIL) vor der Tumoroperation (a) IL-4 und IL-5, jedoch nicht IL-6, oder (b) IL-5, jedoch nicht IL-4 und IL-6, umfasst;
optional umfasst die GBM-Therapie eine Tumoroperation, eine Strahlentherapie sowie die Verabreichung eines Chemotherapeutikums,
- vorzugsweise wird das Chemotherapeutikum oral, intravenös, intraarteriell, intrathekal, durch Infusion über Liquorwege (CSF) einschließlich Shunts und Reservoirs oder durch Implantation eines sich auflösenden Wafers verabreicht,
- vorzugsweise wird das Chemotherapeutikum ausgewählt aus der Gruppe bestehend aus: Temozolomid (TMZ); 1,3-Bis(2-chlorethyl)-1-nitrosoharnstoff (Carmustin); 1-(2-Chlorethyl)-3-cyclohexyl-1-nitrosoharnstoff (Lomustin); gezielte Chemotherapeutika wie 3-(1-Methylindol-3-yl)-4-[1-[1-(pyridin-2-ylmethyl)piperidin-4-yl]indol-3-yl]pyrrol-2,5-Dion (Enzastaurin); Angiogenese-Inhibitoren wie 2-[(2S,5R,8S,11S)-5-Benzyl-11-{3-[(diaminomethyliden)amino]propyl}-7-methyl-3,6,9,12,15-pentaoxo-8-(Propan-2-yl)-1,4,7,10,13-pentaazacyclopentadecan-2-yl]essigsäure (Cilengitid); und Angiogenese/mTOR-Inhibitoren wie (1R,2R,4S)-4-{(2R)-2-[(3S,6R,7E,9R,10R,12R,14S,15E,17E,19E,21S,23S,26R,27R,34aS)-9,27-Dihydroxy-10,21-dimethoxy-6,8,12,14,20,26-hexamethyl-1,5,11,28,29-pentaoxo-1,4,5,6,9,10,11,12,13,14,21,22,23,24,25,26,27,28,29,31,32,33,34,34a-Tetracosahydro-3H-23,27-epoxypyrido[2,1-c][1,4]oxazacyclohentriacontin-3-yl]propyl}-2-methoxycyclohexyl-3-hydroxy-2-(hydroxymethyl)-2-methylpropanoat (Temsirolimus) oder Dihydroxy-12-[(2R)-1-[(1S,3R,4R)-4-(2-Hydroxyethoxy)-3-methoxycyclohexyl]propan-2-yl]-19,30-dimethoxy-15,17,21,23,29,35-Hexamethyl-11,36-dioxa-4-azatricyclo[30.3.1.0]hexatriaconta-16,24,26,28-tetraen-2,3,10,14,20-penton (Everolimus),
- vorzugsweise umfasst die GBM-Therapie ferner eine zusätzliche Immuntherapie, umfassend die Verabreichung eines Immuntherapeutikums, ausgewählt aus der Gruppe bestehend aus: anti-vaskulären endothelialen Wachstumsfaktor (VEGF) Antikörper, vorzugsweise Bevacizumab; Programmed Death-Ligand Inhibitoren (PD-/PD-L1) oder epidermale Wachstumsfaktorinhibitoren, vorzugsweise Gefitinib oder Erlotinib; und Tyrosinkinase-Rezeptor-Inhibitoren, die auf epidermale Wachstumsfaktorrezeptoren abzielen.

3. Ein Verfahren zur Vorhersage der erhöhten Empfänglichkeit eines GBM-Patienten für Immuntherapie, wobei das Verfahren das Bestimmen des Zytokinprofils in einer zuvor erhaltenen Probe des peripheren Blutes, der Liquor cerebrospinalis (CSF), des Knochenmarks oder der Tumorinfiltrierenden Lymphozyten (TIL) des GBM-Patienten vor der Tumoroperation umfasst, wobei die Identifizierung jedes oder keines von entweder:
(a) Interleukin-4(IL-4), Interleukin-5 (IL-5) und Interleukin-6 (IL-6); oder
(b) Interferon Gamma (IFN-γ), Tumornekrosefaktor Alpha (TNF-a) und Interleukin-17A (IL-17A),
im Zytokinprofil des Patienten auf die erhöhte Empfänglichkeit des Patienten für Immuntherapie hinweist.

4. Das Verfahren nach Anspruch 3, wobei die Empfänglichkeit des Patienten für eine Immuntherapie im Vergleich zu GBM-Patienten erhöht ist, deren Zytokinprofil des peripheren Blutes, Liquor cerebrospinalis (CSF), Knochenmarks oder der Tumorinfiltrierende Lymphozyten (TIL) vor der Tumoroperation umfasst: a) IL-4 und IL-5, jedoch nicht IL-6, oder (b) IL-5, jedoch nicht IL-4 und IL-6, vorzugsweise ist die Immuntherapie eine Ergänzung zur GBM-Therapie und umfasst die Verabreichung eines Immuntherapeutikums, ausgewählt aus der Gruppe bestehend aus: einem anti-vaskulären endothelialen Wachstumsfaktor (VEGF) Antikörper, vorzugsweise Bevacizumab; Programmed Death-Ligand Inhibitoren (PD-/PD-L1) oder epidermale Wachstumsfaktorinhibitoren, vorzugsweise Gefitinib oder Erlotinib; und Tyrosinkinase-Rezeptor-Inhibitoren, die auf epidermale Wachstumsfaktorrezeptoren abzielen.

5. Das Verfahren nach einem der Ansprüche 1 bis 4, wobei die Probe eine zuvor erhaltene Vollblutprobe des peripheren Blutes des Patienten ist.

## Revendications

1. Procédé permettant de prédire la survie prolongée d'un patient atteint de glioblastome multiforme (GBM), dans lequel ledit procédé comprend la détermination du profil cytokinique dans un échantillon obtenu au préalable du sang périphérique, du liquide céphalo-rachidien (LCR), de la moelle osseuse ou des lymphocytes infiltrant la tumeur (TIL) du patient atteint de GBM avant une opération de la tumeur, dans lequel l'identification de tous ou d'aucun de :
(a) l'interleukine 4 (IL-4), l'interleukine 5 (IL-5) et l'interleukine 6 (IL-6) ; ou
(b) l'interféron gamma (IFN-γ), le facteur de nécrose tumorale alpha (TNF-a) et l'interleukine 17A (IL-17A),
dans le profil cytokinique dudit patient est indicative de la survie prolongée du patient après une thérapie du GBM.

2. Procédé selon la revendication 1, dans lequel la survie du patient est prolongée par comparaison à la survie de patients atteints de GBM dont le profil cytokinique du sang périphérique, du liquide céphalo-rachidien (LCR), de la moelle osseuse ou des lymphocytes infiltrant une tumeur (TIL) avant une opération de la tumeur comprend certains mais pas tous de :
(a) l'IL-4, l'IL-5 et l'IL-6 ; ou
(b) l'IFN-γ, le TNF-α et l'IL-17A,
éventuellement, la survie du patient est prolongée par comparaison à celle de patients atteints de GBM dont le profil cytokinique du sang périphérique, du liquide céphalo-rachidien (LCR), de la moelle osseuse ou des lymphocytes infiltrant la tumeur (TIL) avant une opération de la tumeur comprend : (a) l'IL-4 et l'IL-5 mais pas l'IL-6 ; ou (b) l'IL-5 mais pas l'IL-4 et l'IL-6,
éventuellement, la thérapie du GBM comprend l'opération de la tumeur, la radiothérapie ainsi que l'administration d'un agent chimiothérapeutique,
- de préférence, l'agent chimiothérapeutique est administré par voie orale, intraveineuse, intra-artérielle, intrathécale, par perfusion via les voies du liquide céphalo-rachidien (LCR) incluant les shunts et les réservoirs, ou par implantation d'une plaquette de dissolution,
- de préférence, l'agent chimiothérapeutique est choisi dans le groupe constitué par : le témozolomide (TMZ) ; la 1,3-bis(2-chloroéthyl)-1-nitrosourée (carmustine) ; la 1-(2-chloroéthyl)-3-cyclohexyl-1-nitrosourée (lomustine) ; les agents chimiothérapeutiques ciblés tels que la 3-(1-méthylindol-3-yl)-4-[1-[1-(pyridin-2-ylméthyl)pipéridin-4-yl]indol-3-yl]pyrrole-2,5-dione (enzastaurine) ; les inhibiteurs de l'angiogenèse tels que l'acide 2-[(2S,5R,8S,11S)-5-benzyl-11-{3-[(diaminométhylidène)amino]propyl}-7-méthyl-3,6,9,12,15-pentaoxo-8-(propan-2-yl)-1,4,7,10,13-pentaazacyclopentadécan-2-yl]acétique (cilengitide) ; et les inhibiteurs de l'angiogenèse/mTOR tels que le 3-hydroxy-2-(hydroxyméthyl)-2-méthylpropanoate de (1R,2R,4S)-4-{(2R)-2-[(3S,6R,7E,9R,10R,12R,14S,15E,17E,19E,21S,23S,26R,27R,34aS)-9,27-dihydroxy-10,21-diméthoxy-6,8,12,14,20,26-hexaméthyl-1,5,11,28,29-pentaoxo-1,4,5,6,9,10,11,12,13,14,21,22,23,24,25,26,27,28,29,31,32,33,34,34a-tétracosahydro-3H-23,27-époxypyrido[2,1-c][1,4]oxazacyclohentriacontin-3-yl]propyl}-2-méthoxycyclohexyle (temsirolimus) ou la dihydroxy-12-[(2R)-1-[(1S,3R,4R)-4-(2-hydroxyéthoxy)-3-méthoxycyclohexyl]propan-2-yl]-19,30-diméthoxy-15,17,21,23,29,35-hexaméthyl-11 ,36-dioxa-4-azatricyclo[30.3.1.0hexatriaconta-16,24,26,28-tétraène-2,3,10, 14,20-pentone (évérolimus),
- de préférence, la thérapie du GBM comprend en outre une immunothérapie d'appoint comprenant l'administration d'un agent immunothérapeutique choisi dans le groupe constitué par : un anticorps anti-facteur de croissance endothélial vasculaire (VEGF), de préférence le bévacizumab ; les inhibiteurs du ligand de mort programmée (PD-/PD-L1) ou les inhibiteurs du facteur de croissance épidermique, de préférence le géfitinib ou l'erlotinib ; et les inhibiteurs des récepteurs de la tyrosine kinase ciblant les récepteurs du facteur de croissance épidermique.

3. Procédé permettant de prédire une sensibilité accrue des patients atteints de GBM à l'immunothérapie, dans lequel ledit procédé comprend la détermination du profil cytokinique dans un échantillon obtenu au préalable du sang périphérique, du liquide céphalo-rachidien (LCR), de la moelle osseuse ou des lymphocytes infiltrant la tumeur (TIL) du patient atteint de GBM avant une opération de la tumeur, dans lequel l'identification de tous ou d'aucun de :
(a) l'interleukine 4 (IL-4), l'interleukine 5 (IL-5) et l'interleukine 6 (IL-6) ; ou
(b) l'interféron gamma (IFN-γ), le facteur de nécrose tumorale alpha (TNF-a) et l'interleukine 17A (IL-17A),
dans le profil cytokinique dudit patient est indicative de la sensibilité accrue du patient à l'immunothérapie.

4. Procédé selon la revendication 3, dans lequel la sensibilité du patient à l'immunothérapie est accrue par comparaison à celle de patients atteints de GBM dont le profil cytokinique du sang périphérique, du liquide céphalo-rachidien (LCR), de la moelle osseuse ou des lymphocytes infiltrant les tumeurs (TIL) avant une opération de la tumeur comprend : (a) l'IL-4 et l'IL-5 mais pas l'IL-6 ; ou (b) l'IL-5 mais pas l'IL-4 et l'IL-6, de préférence ladite immunothérapie est complémentaire à la thérapie du GBM et comprend l'administration d'un agent immunothérapeutique choisi dans le groupe constitué par : un anticorps anti-facteur de croissance endothélial vasculaire (VEGF), de préférence le bévacizumab ; les inhibiteurs du ligand de mort programmée (PD-/PD-L1) ou les inhibiteurs du facteur de croissance épidermique, de préférence le géfitinib ou l'erlotinib ; et les inhibiteurs des récepteurs de la tyrosine kinase ciblant les récepteurs du facteur de croissance épidermique.

5. Procédé selon l'une quelconque des revendications 1 à 4, dans lequel l'échantillon est un échantillon de sang total obtenu au préalable de sang périphérique dudit patient.


### SEQUENCE LISTING

<110> polybiocept GmbH
<120> Methods for predicting prolonged survival and increased treatment susceptibility of glioblastoma multiforme (GBM) patients
<130> 7027/P/1010-EP
<160> 7
<170> PatentIn version 3.5
<210> 1
   <211> 15
   <212> PRT
   <213> Homo sapiens
<400> 1
   Thr Leu Gly Glu Phe Leu Lys Leu Asp Arg Glu Arg Ala Lys Asn
   1               5                   10                  15
<210> 2
   <211> 20
   <212> DNA
   <213> Homo sapiens
<400> 2
   atgggtgccc cgacgttgcc 20
<210> 3
   <211> 20
   <212> DNA
   <213> Homo sapiens
<400> 3
   gcagctccgg ccagaggcct 20
<210> 4
   <211> 15
   <212> PRT
   <213> Homo sapiens
<400> 4
   Ala Arg Gly Pro Glu Ser Arg Leu Leu Glu Phe Tyr Leu Ala Met
   1               5                   10                  15
<210> 5
   <211> 13
   <212> PRT
   <213> Homo sapiens
<400> 5
   Leu Glu Glu Lys Lys Gly Asn Tyr Val Val Thr Asp His
   1 5 10
<210> 6
   <211> 15
   <212> PRT
   <213> Homo sapiens
<400> 6
   Glu Phe Tyr Leu Ala Met Pro Phe Ala Thr Pro Met Glu Ala Glu
   1 5 10 15
<210> 7
   <211> 15
   <212> PRT
   <213> Homo sapiens
<400> 7
   Ser Leu Leu Met Trp Ile Thr Gln Cys Phe Leu Pro Val Phe Leu
   1 5 10 15